Europäisches Patentamt

European Patent Office

Office européen des brevets

(11) Publication number: 0 268 366 B1

# EUROPEAN PATENT SPECIFICATION

(45) Date of publication of patent specification: 04.01.95

(51) Int. Cl.⁶: **A61N 1/32**, A61N 1/34, A61N 1/36

(21) Application number: 87308775.3

(22) Date of filing: 02.10.87

(54) **Methods of and apparatus for generating low frequency electrical stimulus signals.**

(30) Priority: 04.10.86 JP 236700/86
08.10.86 JP 239632/86
08.10.86 JP 239633/86
08.10.86 JP 239634/86
08.10.86 JP 239635/86
08.10.86 JP 239637/86
08.10.86 JP 239638/86

(43) Date of publication of application:
25.05.88 Bulletin 88/21

(45) Publication of the grant of the patent:
04.01.95 Bulletin 95/01

(84) Designated Contracting States:
AT BE CH DE ES FR GB GR IT LI LU NL SE

(56) References cited:
EP-A- 0 204 525          WO-A-86/02567
DE-A- 3 207 050          US-A- 2 820 453
US-A- 3 449 768          US-A- 3 490 458
US-A- 4 431 000          US-A- 4 664 117

(73) Proprietor: **TOTAL HUMAN MEDICAL LABORA-TORY CO., LTD.**
**No. 12-7, Kamiosaki 2-chome**
**Shinagawa-ku**
**Tokyo 141 (JP)**

(72) Inventor: **Anzai, Hiroshi Total Human Medical**
**Laboratory Co., Ltd.**
**No. 12-7, Kamiosaki 2-chome**
**Shinagawa-ku**
**Tokyo 141 (JP)**
Inventor: **Futsuki, Atsunori Total Human Medi-cal**
**Laboratory Co., Ltd.**
**No. 12-7, Kamiosaki 2-chome**
**Shinagawa-ku**
**Tokyo 141 (JP)**

(74) Representative: **Pilch, Adam John Michael et al**
**D. YOUNG & CO.,**
**21 New Fetter Lane**
**London EC4A 1DA (GB)**

## Description

The present invention relates to methods of and apparatus for generating low frequency electrical stimulus signals, which signals may be used to provide an electrical stimulus to a biological body.

It has been proposed that, when audible stimulus is given to a biological body by, for example, playing suitable music such as light or classical music, curative effects for pain such as shoulder discomfort or for other unpleasant feelings can be achieved since the biological body feels pleasant or unpleasant based upon the experimental conception, or its attention is forcibly concentrated on to matters other than pain and unpleasant feelings. Similarly, it is also known that when an electrical stimulus (such as skin or percutaneous stimulus) is given to a biological body, pain, shoulder discomfort or other unpleasant feelings can be relaxed or cured.

According to experimental ideas, it has also been proposed that a biological body can feel increased refresh stimulus, which can achieve higher curative effects, when a relatively frequent stimulus is given to the biological body rather than a stimulus which is provided at a lower rate, and also that a stimulus regularly given to the biological body is better than an irregular stimulus.

Based upon the above-described observations and proposals, various curing methods have been proposed to reduce pain.

As one example of an electrical stimulus signal generating technique, there has been proposed a method of producing an electrical stimulus signal, the pulse repetition frequency of which is not varied in accordance with a lapse in time, and for giving such a stimulus signal to a biological body, in particular to sympathetic nerves and parasympathetic nerves. Moreover, an electrical stimulus signal generating apparatus has been proposed, for example, In Japanese Patent Application Publication No. 56-5543, 1981 (hereinafter referred to as "the first reference") and Japanese Utility Model Publication No. 56-22921 (hereinafter referred to as "the second reference").

The first reference describes a technique in accordance with the so-called 1/f fluctuation theory, in which a musical signal is analyzed as a frequency spectrum, the power spectrum density of the analyzed signal being inversely proportional to the frequency, while the frequency of the electrical stimulus signal is changed over a relatively long time period from 0.5 to 4 seconds within a frequency range between 10 and 100 Hz, and an electrical stimulus signal is thereby produced.

On the other hand, the second reference describes a technique in which the pulse repetition frequency of the electrical stimulus signal and also an irregular pulse pattern (in which the generating duration time of the same pulse repetition is varied) are previously recorded on a recording medium, and the reproduction of the recorded data from the recording medium is performed in accordance with the 1/f fluctuation theory.

Since, as has been found in this field, when the tempo of music is synchronized with the nerves and further with tissue and cells, the effects of pain reduction can be enhanced. Accordingly, this effect can be realized in that, if the sound volume level (or sound pressure level) is used as sound information, the percutaneous stimulus can be given to a biological body in synchronism with the tempo of the music.

However, since according to the above-described technique, the percutaneous stimulus is given to a biological body in accordance with the frequency as derived by the 1/f fluctuation theory, it is practically difficult to fit this stimulus with the tempo of the music.

Moreover, it is known in this field that when an audible stimulus such as music or other sounds and a percutaneous stimulus (electrical stimulus) reflecting the audible information are combined and provided to a biological body, a greater curative effect in reducing pain can be expected as compared with a single stimulus. The curative effect may be furthermore emphasized when both the audible stimulus and the electrical stimulus are provided to the biological body in substantially real time.

However, according to the above-described techniques, neither method can provide both stimuli to a biological body in real time. Accordingly, since the electrical stimulus is given to a biological body even when the music is under a rest condition or muted, thereby providing lower sound pressure, this stimulus is not matched with the experimental pain reduction of a biological body. As a result, the biological body experiences this stimulus as continuous variations of mere stimulus patterns, so that no significantly-increased curative effects can be expected to lead to reduced pain.

US Patent No. US-A-2 820 453 discloses a technique for applying electrical impulses to a patient for therapeutic purposes. In one arrangement, a transducer feeds two electrical channels, one of which is an audio channel capable of providing sound such as music via a loudspeaker; the other is a "shocking" channel for providing electrical shock impulses of between 100 and 200 volts. It is proposed that the audio channel may provide a recorded music selection with shocking impulses being experienced by the patient when the music is of a desired intensity.

According to a first aspect of the present invention there is provided low frequency electrical stimulus signal generating apparatus for generating a stimulus signal for a patient from a sound source such that the patient may simultaneously hear sound from the sound source and feel the stimulus signal, the apparatus being characterised by:

an input section for setting and adjusting operating conditions of the apparatus;

a waveform control section operable to output a signal with a low frequency component of the sound from the sound source;

a control section operable to sample the sound volume level from the sound source at a rate between 2 and 100 Hz, and to convert the sampled sound levels into signals of respective frequencies below 60 Hz; and

a current and/or voltage control section responsive to a selected one of the low frequency sound component and the respective frequencies from the control section depending on the setting of the input section to produce current and/or voltage stimulus signals modulated by the selected one of the low frequency sound component and the respective frequencies.

According to a second aspect of the present invention there is provided a method of generating a low frequency electrical stimulus signal for a patient from a sound source such that the patient may simultaneously hear sound from the sound source and feel the stimulus signal, the method being characterised by the steps of:

deriving a low frequency component of the sound from the sound source;

sampling the sound volume level from the sound source at a rate between 2 and 100 Hz;

converting the sampled sound levels into respective frequencies below 60 Hz; and

producing current and/or voltage stimulus signals modulated by a selected one of the low frequency sound component and the respective frequencies.

Preferably, the respective sound levels obtained by the sampling operation may be converted into pulse number control values, and finally pulse number modulation is carried out in accordance with these pulse number control values so as to effect the current or voltage control.

In a preferred embodiment of the invention, the pulse number modulation is performed in such a manner that the pulse number is within a range from 0 to 30 in response to the sound volume level. The stimulus generating apparatus preferably includes a control system selecting section for switching only one of the current and voltage stimulus signals based upon control information derived from the control section and for outputting the same as the electrical stimulus signal.

Moreover, the current control section may include a waveform generating section for outputting a voltage waveform signal corresponding to the sound volume levels, and a current stimulus signal generating circuit for outputting a current stimulus signal having the same waveform as that of an input voltage waveform signal.

In addition, the control section may include a waveform generating section for generating a voltage waveform signal having a frequency corresponding to the sound volume level, and a voltage stimulus signal generating circuit for outputting the input voltage waveform signal as a voltage stimulus signal.

In the preferred embodiment, the control section includes means for subdividing the sound volume levels into a plurality of level steps, a read-only-memory for previously storing a sound volume level-to-frequency conversion table, and means for reading out from the read-only-memory a frequency corresponding to the sound volume level at each sampling operation.

The sampled sound volume level is preferably set to a zero level when the detected sound volume level is equal to zero or is lower than a minimum level.

In accordance with the preferred embodiment, the current or voltage controlling is performed based upon the sound pressure level of the sound information, so as to modulate the electrical stimulus signal in an irregular manner. Accordingly, the patient can be stimulated by electrical stimulus in response to the tempo of the sound information. Also, no stimulus time period can be made with the hearing stimulus when the music is in a rest or muted condition, or the sound volume level is low. As a result, percutaneous stimulus is given to the patient at the timing at which the patient senses the stimulus in substantially real time, and thus the curing effects of a musical cure using this technique can be improved as compared with the previously-proposed curing effects.

According to the preferred embodiment, since the sound pressure sampling is performed with a shorter cycle by which the patient feels in substantially real time, the electrical stimulus can be modulated in substantially real time with the hearing stimulus, so that unpleasant feelings are removed and the curative effects to reduce pain can also be improved.

In addition, the frequency of the electrical stimulus signal is selected to be a low frequency between 0 and 60 Hz, so that the mental and physical reactions of the patient can be relaxed by a pulling phenomenon against the natural vibration of automatic nerve reaction (for instance, in the so-called phenomena of "KEDACHI (goose flesh)" and "FURUE (tremble)" in order to provide a relaxed feeling to the patient, the natural vibration of which is approximately 15 Hz.

In the preferred embodiment, a judgement is first made whether or not sound information is present. If sound information is present, only low frequency information having a frequency lower than approximately 200 Hz is derived. As a result of the detection, when no sound information is detected, quasi-sound information having an arbitrary waveform is generated from a rhythm generator which may be provided independently. The current or voltage control is performed by utilizing the respective waveforms of the low frequency sound information and quasi-sound information. It is preferable that the intensity of the electrical stimulus signal is selectively adjustable based upon the information supplied from the external source.

The waveform control section preferably includes a comparator for detecting whether or not sound information is present, a low pass filter for filtering low frequency sound information only having a frequency lower than approximately 200 Hz, a rhythm generator for generating quasi-sound information, and a selection switch for selecting the low frequency sound information when sound information is input, and the quasi-sound information when sound information is not input.

In the preferred embodiment, the current control section includes an intensity setting circuit of the rhythm-modulation waveform signal, and a current stimulus signal generating circuit for outputting a current stimulus signal having the same waveform as that of a waveform input from the intensity setting circuit, and further the voltage control section includes a voltage stimulus circuit for outputting a voltage stimulus signal by adjusting the rhythm-modulated waveform signal.

In the preferred embodiment, the intensity of the electrical stimulus signal is selectively adjustable.

Either current control or voltage control may be performed based upon the waveform of the sound information so as to modulate the electrical stimulus signal in an irregular manner. As a result, the patient can be electrically stimulated, in other words, a percutaneous stimulus can be provided, in accordance with the tempo of the music or the like. When there is no sound information, the patient can be stimulated by the waveforms of the quasi-sound information which has been generated. Consequently, since the patient can be stimulated with the percutaneous stimulus at the rate at which it can be sensed in substantially real time, reduction in pain and curative effects can be further improved, as compared with the previously-proposed musical medical treatments.

Furthermore, since only the sound information is utilized which contains low frequencies (lower than about 200 Hz) typically of the bass and drum, unpleasant feelings can be effectively eliminated.

In the preferred embodiment, the control section includes means for subdividing the sound volume levels into a plurality of level steps, and means for converting the subdivided levels into the corresponding amplitude control values and for outputting the converted control values.

Moreover, in the preferred embodiment, an electrical stimulus signal having 24 steps is obtained as the amplitude control value within a range between 0 and 100% of the initial setting intensity.

In another mode of operation, control information previously stored in the control section may be read out without using acoustic information from the sound source so as to control the current and voltage stimulus signals, and the current and voltage stimulus signals are alternately output as the electrical stimulus.

The control information is preferably selected from more than one sort of waveform, frequency and intensity information. The control information may be selectable and variable to appropriate values suitable for the patient in response to external input.

Moreover, the alternating operations of the current and voltage stimulus signals may be performed at random.

The time interval between the succeeding alternating operations is preferably settable at random within a range from several seconds to several tens of seconds. In particular, the alternating time interval is preferably selected to be approximately 2 seconds to 1 minute.

Furthermore, the frequency of the electrical stimulus signal is preferably selected to be a low frequency signal at a frequency between 0 and 60 Hz.

The invention will now be described by way of example with reference to the accompanying drawings, throughout which like parts are referred to by like references, and in which:

Figure 1 is a block diagram illustrating a basic arrangement according to an embodiment of the present invention;

Figures 2A and 2B are block diagrams, each of which shows a portion of a detailed block diagram of a low frequency electrical stimulus signal generating apparatus according to an embodiment of the present invention;

Figures 3A to 3K are voltage waveform signal diagrams of signals appearing in the circuit of Figures 2A and 2B;

Figure 4 is a schematic diagram for explaining a table conversion;

Figure 5 is a functional block diagram of a CPU shown in Figure 2A;

Figures 6 to 14 are flowcharts of operations according to embodiments of the present invention.

Figure 15 shows a current stimulus circuit;

Figure 16 shows a voltage stimulus circuit;

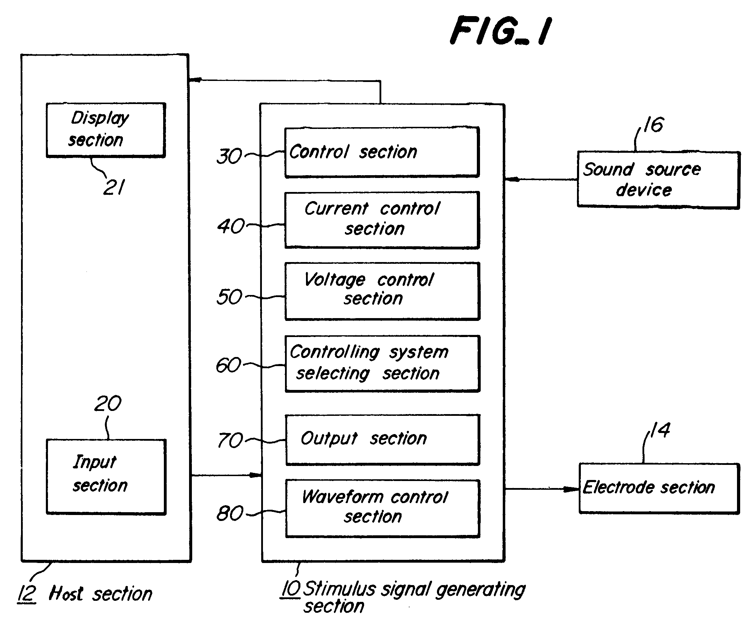

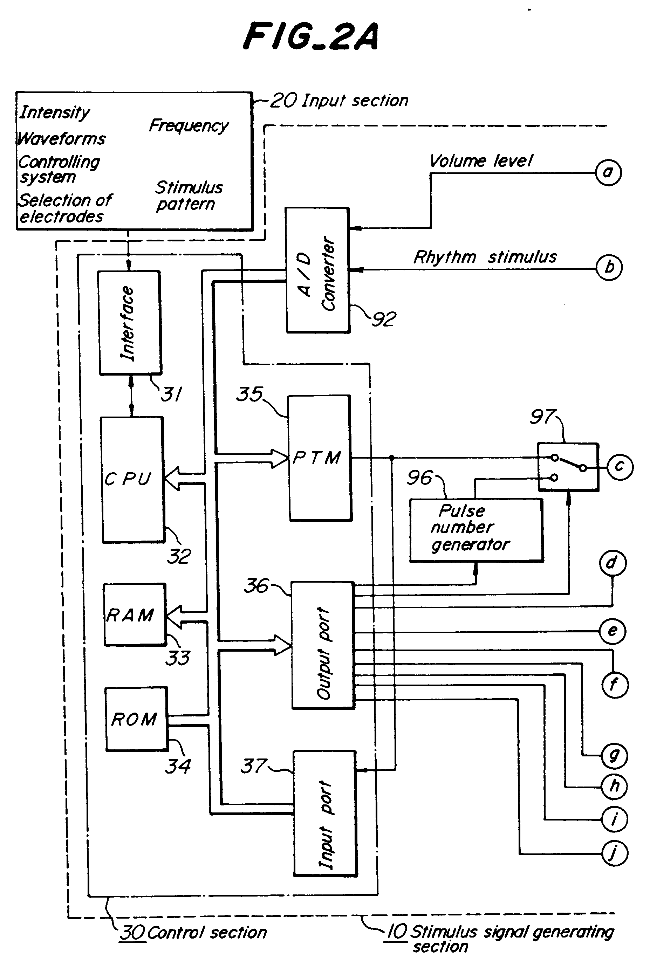

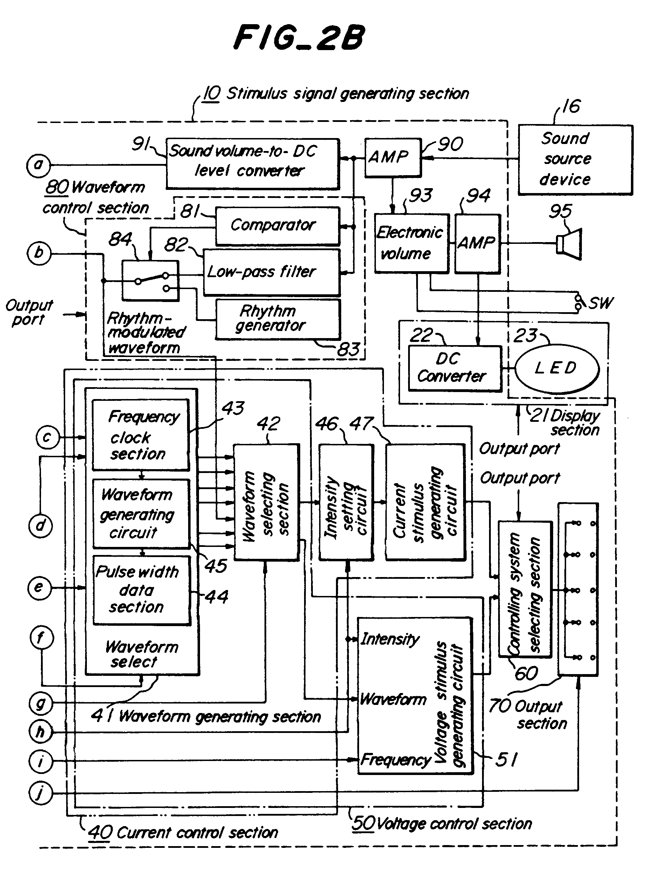

Figure 1 is a block diagram illustrating a basic arrangement for explaining aspects of the present invention, while Figures 2A and 2B are block diagrams each of which illustrates a portion of a detailed block diagram of a low frequency electrical stimulus signal generating apparatus embodying the present invention. In Figures 2A and 2B, portions indicated by the same reference marks "a" to "j" should be connected to each other.

Referring now to Figure 1, there is shown a low frequency electrical stimulus signal generating apparatus including a stimulus signal generating section 10 for generating a low frequency electrical stimulus signal, a host section 12 for selectively supplying data required to drive the stimulus signal general generating section 10, an electrode section 14 for applying the generated electrical stimulus signal to a biological or living body and, if required, a sound source device 16.

In the preferred embodiment, the host section 12 includes an input section 20 for inputting various types of signal to the stimulus signal generating section 10. These signals are to set the initial or predetermined conditions, to adjust the required conditions, or to initialize the generation of the electrical stimulus signal. The above-described predetermined conditions include, for instance, the amplitudes (or intensity), the waveforms, the frequencies, the stimulus patterns, controlling systems for controlling current or voltage, and selection of electrodes. In addition, if required, the host section 12 may further include a sound source device for generating music or other sounds, an electric converting or transducer device, such as a speaker or earphone for reproducing the acoustic information originating from the sound source device, and/or a display section 21 for visually displaying the generated electrical stimulus signals. Alternatively, instead of provision of a sound source device in the host section 12, it may be connected to the stimulus signal generating section 10 as an external sound source device 16.

In the present embodiment, the stimulus signal generating section 10 employs a current or voltage control system. According to this system, the volume of the sound level supplied from the sound source device 16 is detected, and the physical quantities of the current or voltage stimulus signals such as frequencies, amplitudes (intensity) and pulse rate or shape are controlled in response to the detected volume levels, while these electrical stimulus signals are continuously modulated; thus modulated electrical stimulus signals are output from the stimulus signal generating section 10. In this case, the electrical stimulus signals are preferably selected in such a manner that the frequency of the stimulus signals is a low frequency within the frequency range of approximately 0 to 50 Hz, at which biological bodies such as patients feel pleasant. To this end, the stimulus signal generating section 10 includes a control section 30 having a CPU (central processing unit) for converting the volume level for controlling the physical quantities into predetermined controlling quantities, and for effecting other desired controls; a current control section 40 for controlling the current in response to the above-defined controlling quantities; a voltage control section 50 for controlling the voltage in response to the above-defined controlling quantities; a controlling system selection section 60 for selecting either the voltage stimulus signal generated from the voltage control section 50, or the current stimulus signal produced from the current control section 40; and an output section 70.

Moreover, in the preferred embodiment, the stimulus signal generating section 10 includes a waveform control section 80 for performing rhythmic stimulus (also referred to as "rhythmic modulation") by controlling the rhythm of the electrical stimulus signal in such a manner that the waveforms of both the current and voltage are controlled by a sound waveform less than approximately 200 Hz during presence of the sound, and by a quasi-sound waveform produced during absence of the sound, independently of the volume level.

In addition, according to the preferred embodiment, the control section 30 includes means for generating various control signals for waveforms, pulse widths, frequencies, intensity (amplitudes) and changing over as parameters to stimulate biological bodies in order to alternately change, at random, the current control system or voltage control system, independently of the music or other acoustic information. As will be discussed later, the pattern of the current or voltage stimulus signal is preset by this control means to the stimulus pattern suitable for the respective biological body.

Referring to Figures 2A and 2B, a description will now be given of the arrangements and also the controlling system. It should be noted that the same reference numerals will be employed to designate the same circuit arrangements as those shown in Figure 1.

In Figures 2A and 2B, the acoustic information from the sound source device 16, e.g. a radio, a television or other acoustic device, is transferred via an amplifier 90 to a sound volume-to-DC level converter 91. The resultant volume level signal is fed to an A/D (analogue-to-digital) converter 92.

The output of the amplifier 90 is also connected to an electric-acoustic converter or transducer 95, such as a speaker or an earphone, via an electronic volume control 93 which can be adjusted by an external switch SW, and an amplifier 94. This output is reproduced and provided to the biological body as a hearing or audible stimulus. It should be noted that the sound reproduction system is not necessarily employed in this electrical stimulus apparatus, but may be substituted by the reproduction system of the external sound source device 16.

The output of the amplifier 94 may be displayed by a light emitting display section 21 in such a manner that variations in the acoustic information are displayed. In this case, the output is processed in a DC converter 22 of the display section 21 which also includes a light emitting diode (LED) 23. It should also be noted that the light emitting display section 21 may be provided within the host section 12.

In the preferred embodiment, the control section 30 includes a series interface 31 for receiving the signal sent from the input section 20 of the host section 12 by way of a wireless or wire system, a central processing unit (CPU) 32 for performing various processes in response to the signal from the interface 31, a random access memory (RAM) 33 for reading and writing data required for performing the various processes in the CPU 32, a read only memory (ROM) 34 for reading out the necessary data which have previously been stored therefrom for processing in the CPU 32, a programmable time unit (PTM) 35 for automatically outputting arbitrary frequencies as an internal clock with respect to the data set by the CPU 32, an output port 36 for outputting the outputs containing various control signals from the control section 30 to other circuit arrangements, and an input port 37 for receiving an input to the control section 30, such as a signal to detect the end of one cycle of the frequency from the PTM 35.

A portion of the output derived from the output port 36 is supplied as a control signal to a pulse number generator 96, and a changing switch 97 for changing the signals from the PTM 35 and the pulse number generator 96.

In the preferred embodiment, both the current control section 40 and the voltage control section 50 commonly employ a waveform generating section 41 and a waveform selecting section 42 comprising, for instance, an electronic switch for selectively deriving the generated voltage waveform. A frequency or pulse number control signal is transferred to a frequency clock section 43 of this waveform generating section 41 via the changing switch 97, and the frequency control signal from the output port 36 is also supplied thereto. Moreover, data for changing the pulse width of the voltage waveform to be generated is transferred to a pulse width data section of the waveform generating section 41.

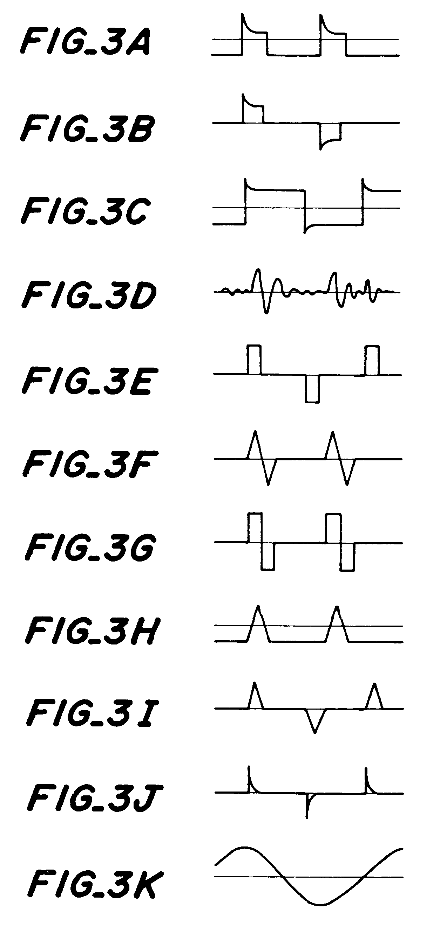

In the waveform generating section 41, there is provided a waveform generating circuit 45. This waveform generating circuit 45 employs plenty of circuits from which various types of voltage waveform signals can be output, as illustrated in Figures 3A to 3K. The frequency of the voltage waveform signal can be controlled by a signal from the frequency clock section 43, and furthermore, the width of the signals can be varied by the data from the pulse width data section 44. It should be noted that such waveform generating circuit 45, frequency clock section 43 and pulse width data section 44 can be easily fabricated by utilizing conventional electronic circuit techniques, and therefore need employ no specific electronic circuit. At least one or more than two of the waveform generating circuits 45 are operated to select one, or more than two sets of the voltage waveform patterns in response to the control signal derived from the output port 36 as a waveform select signal. Also, the waveform generating section 41 is so designed that one or more than two sets of the voltage waveform patterns output from the waveform generation section 41 can be derived based upon a waveform select signal from the output port 36. The circuit arrangement of the waveform selecting section 42 may also be manufactured by employing conventional circuit techniques, and requires no specific circuit arrangement.

One of the voltage waveform signals selected by this waveform selecting section 42 is supplied to a current stimulus generating circuit 47 via an intensity (amplitude) setting circuit 46 of the current control section 40. The other of the voltage waveform signals is supplied to a voltage stimulus generating circuit 51 of the voltage control section 50.

In the current control section 40, the voltage waveform signal input into the intensity setting circuit 46 is set to a voltage waveform signal having a suitable amplitude in response to the control signal from the output port 36, whereas in the current stimulus generating circuit 47, its input voltage waveform pattern is converted into a current waveform type electrical stimulus signal having the same pattern as the voltage waveform pattern.

In, on the other hand, the voltage control section 50, the voltage waveform signal input into a supply voltage stimulus generating circuit 51 via the waveform selecting section 42, is converted into a voltage waveform type electrical stimulus signal having a particular intensity and frequency in response to an intensity (amplitude) and frequency control signal from the output port 36.

In addition, in the preferred embodiment, there is a controlling system selecting section 60 for selecting the electrical stimulus signal produced in accordance with either the current control system, or the voltage control system, and for supplying the selected electrical stimulus signal to a biological body as skin stimulus. This selecting operation can be performed in response to a selecting system control signal derived from the output port 36, whereby either the current control system, or the voltage control system is selected; otherwise, both control systems are alternately selected.

The electrical stimulus signal obtained via this controlling system selecting section 60 is transferred to an output section of the low frequency electrical stimulus signal generating apparatus. To this output section 70, electrodes for applying skin stimulus to more than one portion of the biological body are connected. A selection of the electrodes is under the control of an electrode selection controlling signal derived from the output port 36.

In the preferred embodiment, the waveform control section 80 includes: means for detecting acoustic information from the amplifier 90, for instance, a comparator 81; a low-pass filter 82 for passing therethrough acoustic information having a frequency lower than approximately 200 Hz; a rhythm generator 83 for generating a quasi-acoustic waveform during a rest or mute period of the acoustic information; and a selection switch 84 for selecting the acoustic information having a frequency lower than approximately 200 Hz supplied from the low-pass filter 82 when the acoustic or sound information is detected by the comparator 81, and for transferring the quasi-acoustic information as a rhythm-modulated (stimulus) waveform to the waveform selecting section 42 when the comparator 81 detects no acoustic information. The above-described acoustic information is also transferred via an A/D converter 92 to the control section, and is displayed as a rhythmic illustration at the display section 21 under the control of the control signal from the output port.

In the present embodiment, the frequency of the electrical stimulus signal for providing the skin stimulation is selected to be a low frequency. In the preferred embodiment, a frequency range is determined to be approximately 0 to 60 Hz, at which a biological body receives a pleasant sensation. By controlling either the current controlling or the voltage controlling in accordance with the sound volume level of the sound source, the electrical stimulus signal is modulated.

In the case of this current controlling system, cells of a biological body feel electric charges as energy, whereas in the case of the voltage controlling system, a small current is sufficient if the body is to feel the same pleasant sensation as that in the current controlling system, because the voltage is fluctuated. It is known in the art that, according to both controlling systems, a biological body can feel a pleasant sensation.

In the preferred embodiment, the following three modulations are performed.

(1) The electrical stimulus signal is modulated by controlling the frequencies of the current and voltage, amplitude and pulse number, in relation to the sound level.

(2) The electrical stimulus signal is rhythm-modulated by controlling the current and voltage while using proper sound waveforms and quasi-sound waveforms having frequencies lower than about 200 Hz, without utilizing the sound level.

(3) The electrical stimulus signal is stimulus pattern-modulated by controlling the current and voltage patterns based upon a combination of various types of control information (waveforms, frequencies and amplitudes) which is previously set in the control section, without using the sound information.

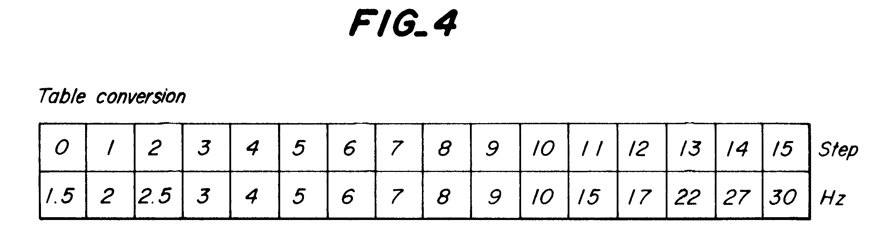

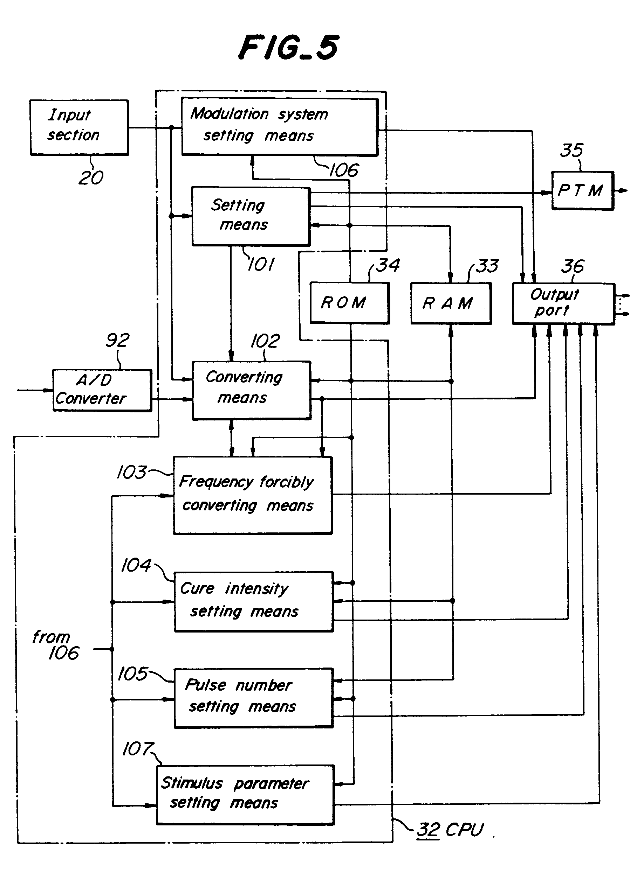

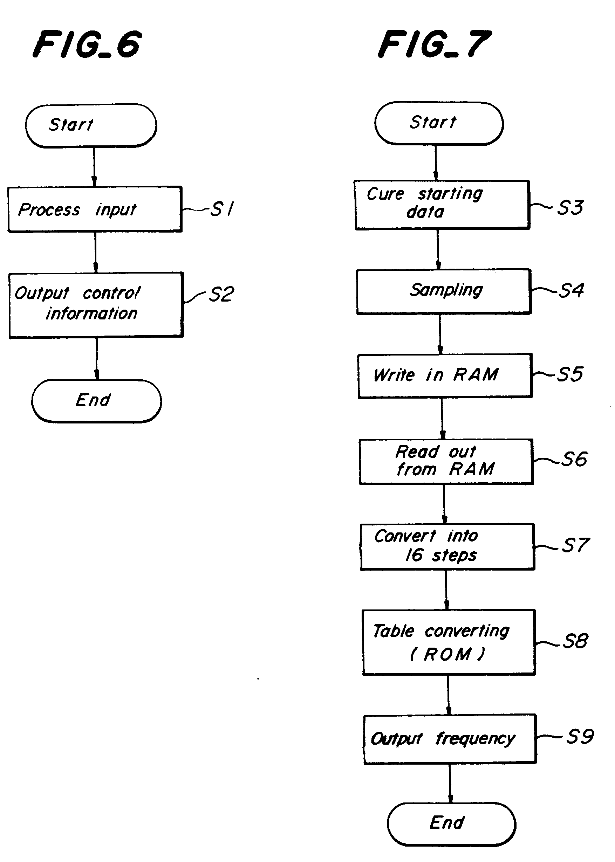

Referring now to Figures 4 to 16, a detailed description will be given. Figure 4 is a schematic diagram for explaining a table conversion. Figure 5 is a functional block diagram of the CPU 32. Figures 6 to 14 are flowcharts of the operations according to the preferred embodiment, where the respective processing steps are indicated by "S".

Sound level frequency modulation will now be described.

First, a power switch is turned on to energize the music source device 16. Thus, acoustic information such as music is produced, thereby giving hearing stimulation to a biological body such as a patient.

The acoustic or sound information originating from the music sound device 16 is converted into a sound level composed of data of suitable bit numbers such as, for example, 8-bit data or 256-bit data, as illustrated in the upper column of Figure 4, by utilizing a sound volume-to-DC level converter 91 and an A/D converter 92 in real time processing.

Predetermined initial information containing the modulation, waveform, frequency, electrode selection, controlling system and so on is input by the input section 20 (e.g. an input key) of the host section 12 to the control section 30 of the stimulus signal generating section 10. Based upon this initial information, the processing is performed by the setting means of the CPU 32 (S1), and corresponding control information is read out from the ROM 34, or converted into predetermined control information. Thereafter, the above-described control information is supplied to the respective circuit arrangement components of the output port 36 to set the initial conditions (S2).

For instance, a waveform select signal is transferred to a waveform generating section 41 to actuate the waveform generating circuit 45, while this control signal is sent to the pulse width data section 44 so as to initial-set the proper pulse width.

When the initial frequency information is sent to the frequency clock section 43, the frequency of the voltage waveform signal from the waveform generating circuit 45 is initial-set in such a manner that a biological body feels the maximum stimulation, for instance, at 30 Hz, when the waveform of the electrical stimulus signal is the same. This initial setting of the frequency is set independently of presence of the sound information supplied from the music sound device 16.

Simultaneously, the control signal is supplied to the voltage controlling and current controlling system selecting section 60 in order to select either controlling system, thereby determining the corresponding electrical stimulus signal.

The intensity of these electrical stimulus signals is set every time the control signal read out from the ROM 34 is transferred to an intensity setting circuit 46 and a voltage stimulus generating circuit 51 in accordance with the selection by the input section 20. This intensity of the electrical stimulus signal can be adjusted until a user feels a pleasant sensation while receiving this electrical stimulus signal.

When this stimulus intensity reaches a desired level, the start input is transported to the control section 30 by turning on the start key of the input section 20 in the host section 12, the medical treatment commencement data is sent to the stimulus signal generating section 10 (S3) while processing in the CPU 32, and then, the control section 30 starts to receive the volume level from the A/D converter 92 (S4). This signal reception is carried out by the sampling operation of the CPU 32. The respective volume levels are once written into the RAM 33, which have been acquired every sampling operation (S5), and are sequentially read out from the RAM 32, if required (S6). The volume level of data of suitable bit numbers such as, for example, 8-bit data or 256-bit data is converted in the CPU 32 into 16-stepped level data (S7).

In this level conversion, not only a zero volume, but also a lower volume is recognized as the electrical stimulus signal having the zero level intensity, which forcibly corresponds to the zero step. The 16-step volume levels are converted into the corresponding frequencies for the lower frequency region (step 8). According to this level conversion, the conversion frequencies corresponding to the respective volume levels are previously stored in the ROM 34 in table form, and thus, the corresponding frequency is read out by comparing it with the frequency conversion table every volume level (S8). This frequency information is sent via the output port 36 to the corresponding circuit arrangement (S9). In this case, the period of sampling is preferably selected to be in a range from approximately 0.01 to 0.5 seconds, for example, at which a biological body senses or feels practically in real time. Also, as the frequency table, the stimulus frequencies from 0 to 60 Hz are allocated to the 16-step volume levels, as illustrated in Figure 4, at which a biological body feels comfortable or pleasant. The frequencies of the stimulus signal are preferably selected to be in a range from 0 to 30 Hz. As is easily understood from Figure 4, the volume level "0" is set to 1.5 Hz, the level "1" is set to 2 Hz, the level "5" corresponds to 5 Hz, the level "10" is equal to 10 Hz, the level "11" is selected to be 15 Hz, ---, the level "15" is selected to be 30 Hz. To carry out such frequency converting, the CPU 32 includes conversion means 102 for performing the frequency conversion by the sampling operation (See Figure 5).

When the volume level is frequency-converted and output from the output port 36 every sampling period, this frequency is supplied to the frequency clock section 43 of the waveform generating section 41 and the voltage stimulus generating circuit 51, whereby the frequencies which have been initially set are sequentially updated by the frequencies newly converted by the received volume level. Under this condition, the current or voltage frequency converting operation is effected, so that the frequency modulation of the electrical stimulus signal can be automatically achieved.

As previously described, music and other sounds are utilized as a hearing stimulus, the sound volume level supplied from the sound source device 16 is sampled in substantially real time in synchronism with the hearing stimulus, and the frequency of the electrical stimulus signal is modulated to the frequency corresponding to the sound volume level at the same time as the sampling time, whereby this electrical stimulus signal is applied to a biological body as a skin or percutaneous stimulus.

The most important aspect of such a frequency modulation is to substantially perform the real-time sampling, and also to set the electrical stimulus (corresponding to the percutaneous stimulus) in response to this sampling operation. In the preferred embodiment, when the sound volume (sound pressure) level is equal to zero, or a very low level (e.g. less than one sixteenth (1/16) of the maximum level), the frequency is set, for instance, to be less than 1.5 Hz. Under such circumstances, the waiting time period for the succeeding stimulus is preset as follows. For instance, for the 1 Hz frequency, the waiting time period is 1 second; for the 0.5 Hz frequency, the waiting time period is 2 seconds; for the 0.25 Hz frequency, the waiting time period is set to 4 seconds. As a result, when using actual music, no percutaneous stimulus is performed during the rest time and lower sound volume conditions. Since no stimulus time of this percutaneous stimulus will in practice be synchronized with no stimulus time period of the hearing stimulus, the cerebrum cortex feels no or reduced pain because the nerve supervision may occur by the experimental concept in the music field, which improves the effects of the cure.

When the waiting time period for the subsequent percutaneous stimulus becomes long, the case may arise where in fact generation of the electrical stimulus signal does not reflect the hearing stimulus. To avoid such a problem, in the preferred embodiment, the control section 30 of the stimulus signal generating section 10 includes frequency forcibly converting means 103 for forcibly converting the input signal to a new frequency corresponding to the newly sampled volume level, when the waiting time period becomes longer than a predetermined level, while continuously monitoring the converting frequency and the sound volume level.

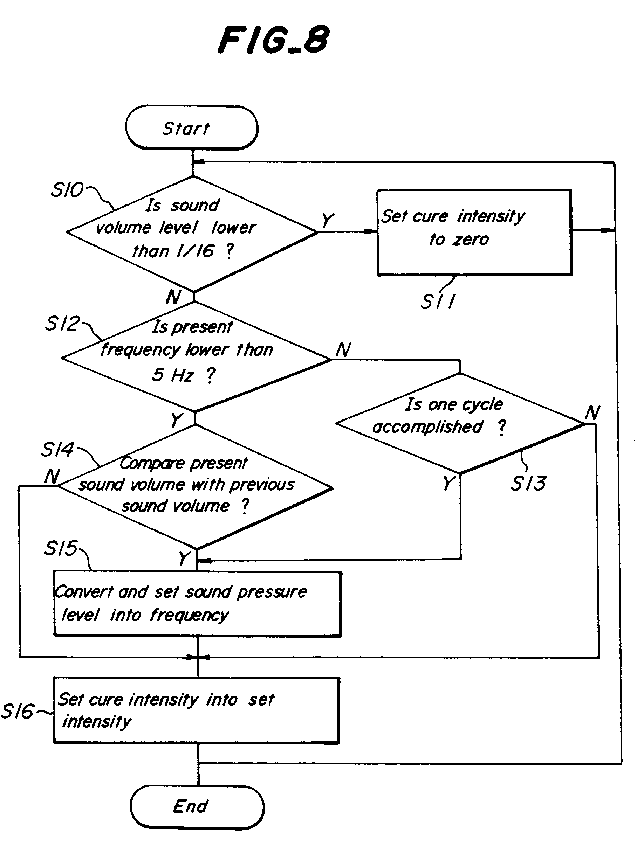

Operation flows of the frequency forcibly converting means 103 will now be described with reference to Figure 8.

At first, a judgement is carried out whether or not the sampled volume level is lower than one sixteenth of the maximum level (S10). If the answer is NO, another judgement is performed whether or not the present frequency is lower than, for instance, 5 Hz (S12). If the answer is YES, that is, if the sound volume level is lower than one sixteenth of the maximum level, the cure intensity is set to the zero step (S11). Then, if the frequency is higher than 5 Hz, a judgement is made whether or not one cycle of this frequency is completed (S13). If one cycle of the frequency is not yet completed, the cure intensity is set to the setting intensity (i.e., the initial setting intensity) (S16). When one cycle of this frequency is accomplished, the volume level is converted into the frequency corresponding to the volume level which has been acquired in the succeeding sampling operation (S15). The resultant signal is output via the step S16. When the present frequency is lower than 5 Hz which has been judged in the above step S13, it is compared with the secound volume level obtained at the previous sampling operation occurring just before the present sampling operation (S14). If the present volume level is higher than the previous volume level, it is forcibly set to the frequency corresponding to the present volume level during the signal process in the step S15, and thereafter processed in the step S16 to output the resultant signal. On the contrary, if the present volume level is lower than the comparison level, the process of the step S16 is carried out. The above-described overall signal process is continuously performed during the operation of the electrical stimulus signal generating apparatus. It should be noted that although the converting means 102 and the frequency forcibly converting means 103 are separately employed in the preferred embodiment, the frequency forcibly converting means 103 may be included as part of the converting means 102. In addition, the operation flow is not limited to the above-described flow.

In order to effect amplitude modulation, at first, an initialization is performed in a similar manner to that effected for frequency modulation (see Figures 5 and 6). In this embodiment, the initialized intensity corresponds to the maximum intensity by which a biological body feels maximum stimulus. This initialized intensity is equal to a set intensity which is written in the RAM 33 and read therefrom, if required.

When, for example, an 8-bit data sound volume level is sequentially taken in by the sampling operation, the set intensity is sequentially updated in real time by another intensity (amplitude) corresponding to the above sound level in a range from 0 to 100 percent (0 - 100%). As a result, amplitude modulation for the electrical stimulus signal can be achieved, whereby the intensity modulation for the percutaneous stimulus can also be achieved. This amplitude (intensity) conversion is performed continuously.

This amplitude modulation is mainly performed by employing the cure intensity setting means 104.

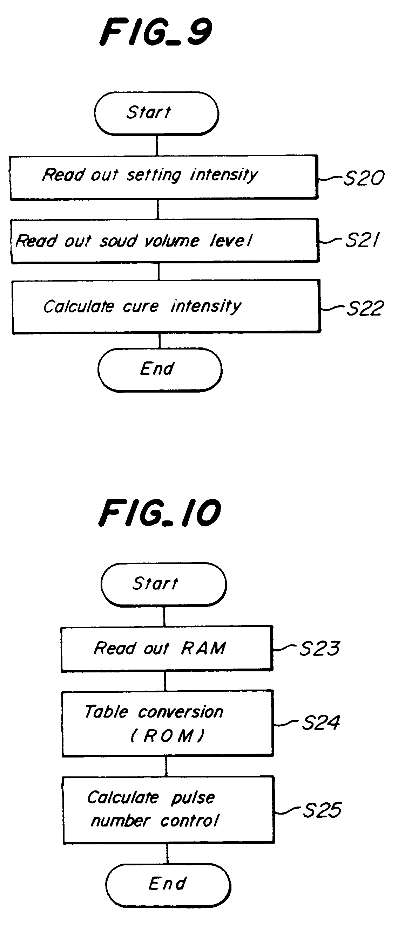

Referring now to Figure 9, the amplitude modulation process will be described. First, the set intensity is read out from the RAM 33 (S20), and secondly, the 8-bit volume level is read out from the RAM 33 (S21). Subsequently, to execute the following equation (I), the 8-bit data of this volume level is divided by 256, and the resultant quotient is multiplied by the set intensity to obtain the cure intensity, that is to say, an amplitude control value (S22). It should be noted that the calculation process is not limited to the above process.

The cure intensity = (the set intensity) x {(the 8-bit data of the sound volume data)/256}     (I).

In this case, the 24-stepped electrical stimulus signal intensity is preferably obtained as the amplitude control value, which is set within a range from 0 to 100 percent. Also, when the music/sound volume level is equal to zero, or very low, the electrical stimulus signal intensity is forcibly set to zero. However, the intensity becomes low when the sound volume level is low, even if this intensity is not set to zero. As a result, a biological body feels no stimulus so that the body sensation is related to the music/sound.

In the preferred embodiment, the calculation process is accomplished in the functional block of the cure intensity setting means 104. It is also possible that the cure intensity (or amplitude control value) is stored as the conversion table in the ROM 34, and this conversion table is read to convert the data into the cure intensity.

The resultant amplitude control value is output from the output port 36, and then transferred to the intensity setting circuit 46 and the voltage stimulus generating circuit 51, where the amplitudes of the current and voltage stimulus signals are modulated. In this case, either the current control system, or the voltage control system is selected by the control system selecting section 60 in the same manner as in the frequency modulation system.

In accordance with this amplitude modulation, the sound volume level supplied from the sound source device is sampled in substantially real time as the hearing stimulus, and amplitude-converted at the same time as the sampling time, so that the amplitude of the electrical stimulus signal is set within a range from 0 to 100 percent of the set intensity in real time.

The main feature of the present amplitude modulation is to supply no electrical stimulus when the music or other sound is in a rest condition and the sound volume is low. That is to say, no stimulus signal of the percutaneous stimulus can be produced in accordance with the music, which causes a significant curing effect in the music curing method.

In order to effect pulse number modulation, similar initialization as for frequency modulation is carried out. Instead of the frequency, a pulse having a pulse rate of, for instance, 16 pulses/second (corresponding to 30 Hz) is produced from the pulse number generator 96 (Figures 5 and 6).

Then, when the 8-bit data of the volume level is sequentially read out, it is sequentially updated in real time by the pulse numbers corresponding to the read volume level within a range of the initialized set pulse number as a maximum pulse number, so that control of the electrical stimulus signal can be achieved, and thus the pulse number control of the percutaneous stimulus can be achieved. This pulse number modulation is continuously performed in the preferred embodiment.

This pulse number modulation is mainly performed by employing the pulse number setting means 105 of the CPU 32.

In accordance with the pulse number modulation process (see Figure 10), the sound volume level subdivided into 16-stepped levels is read out from the RAM 33 (S23), and then the corresponding pulse-number-converted value is obtained from the ROM 34 by the table conversion (S24). Subsequently, the following equation (II) is calculated (S25).

Pulse number control value = (16-stepped converted values) - 1      (II)

In the preferred embodiment, the pulse number control value calculation is based upon the equation (II), and thereafter sent via the output port 36 to the pulse number generator 96. Accordingly, the pulse is generated at the pulse rate corresponding to the sound volume level which has been sampled, and transferred via a changing switch 97 to the frequency clock section 43 in the waveform generating section 41. As a result, the current and voltage stimulus signals can be produced as the pulse signal, the pulse rate of which is controlled. The pulse numbers employed in the preferred embodiment are preferably set in the range from 0 to 30 pulses/second at which a biological body feels a pleasant sensation.

According to the above equation (II), the pulse number becomes zero when music is in the rest condition and the sound volume is low.

It should be noted that the calculation of the pulse number control value is not limited to the above-defined equation (II), but may be realized by different calculation methods. Instead of the equation calculation, the pulse number control value corresponding to the sound volume level is previously stored in the ROM 34, and then, the necessary data may be obtained from the ROM 34 by way of the table conversion.

As has been described in the above preferred embodiment, the percutaneous stimulus can be applied to a biological body with the hearing stimulus in substantially real time by employing pulse number modulation, in similar manner to the frequency modulation.

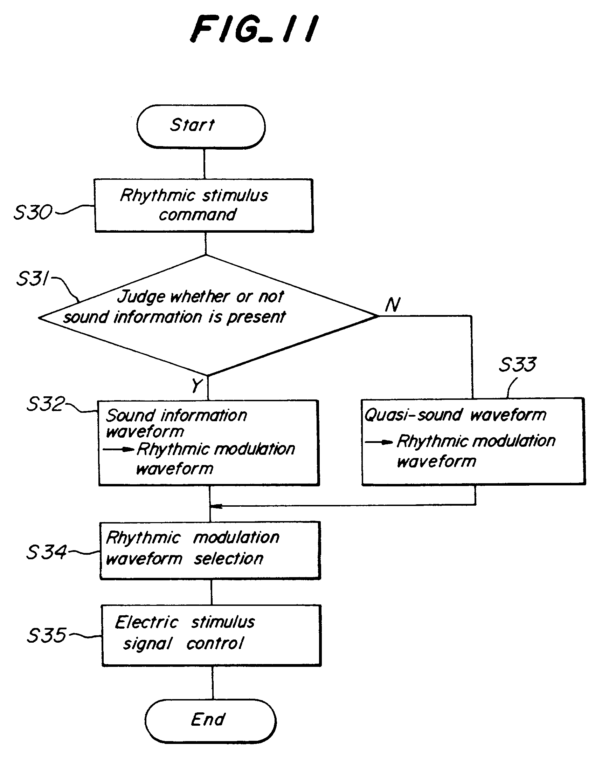

Referring now to Figure 11, a rhythmic modulation process will be described, not using the sound volume level. The setting conditions such as the modulation system, waveform, stimulus pattern, control method, and electrode selection are input into the control section 30 from the input section 20 of the host section 12 as curing data. This processing will now be described. As in the above-described frequency modulation and other modulations, the initial data setting is performed based upon these setting conditions. To sufficiently feel the strong intensity, a waveform having a large wave crest is employed while the initial setting is effected. For example, when a biological body starts to feel, as optimum stimulus, the stimulus pattern, the medical treatment will be commenced. At first, the rhythmic modulation command information is sent from the output port to the waveform control section 80 by the modulation system setting means 106 of the CPU 32, so as to enable this waveform control section 80 (S30). A judgement is made whether or not the sound information supplied from the sound source device 16 is present or not (S31). When the output is present, it is compared by the comparator 81 with, for instance, a set level, and the changing switch 84 is changed over to the low pass filter 82 so as to send sound information of frequency lower than approximately 200 Hz to the waveform selecting section 42 as the rhythmic modulation waveform (S32). When, on the other hand, no sound information is present (S33), in response to the command sent from the modulation system setting means 106, the waveform selection 42 is changed to select only the rhythm-modulation waveform, which is sent to the succeeding stage (S34).

The rhythm-modulated waveform is sent from this waveform selecting section 42 to the voltage stimulus generating circuit 51, and also to the current stimulus generating circuit 47 via the intensity setting circuit 46 (S32). Based upon both the rhythm-modulated waveform, the rhythm and/or intensity of the current and voltage stimulus signal are controlled (S33), and are output as the electrical stimulus signal in accordance with either the current or voltage controlling system, as in the above-described other modulation systems. As a result, rhythmic and intensity stimulus can be applied to a biological body.

In the preferred embodiment, the reason why the low pass filter has a pass band lower than approximately 200 Hz, is that the stimulus which is achieved by the frequency pattern within this frequency range can be derived from the stimulus obtained by the bass and drum sounds in music.

By receiving such rhythmic stimulus, unpleasant sensations of the biological body can be relaxed, mitigated or cured.

In the preferred embodiment, the parameters for stimulus patterns selected by the control section 30 are automatically varied with no reaction to music. Thus stimulus pattern modulation can be achieved without employing a sound source device alternately changing the current or voltage control systems. These parameters typically contain waveforms, pulse widths, frequencies and intensities.

In the present embodiment, two examples where the frequencies and intensities are used as variable parameters, will now be described for the stimulus pattern modulation processing with reference to Figures 2, 5 and 12.

The initial setting values and also the variable parameters values for the frequencies and intensities have been previously stored in the ROM 34.

Firstly, the case will be described in which the frequency is variable in stimulus pattern modulation processing.

In response to commands sent from the input section 20, the stimulus parameter modulation is designated by a modulation system setting means 106, as in the previous modulation system (S40). Then, the initial setting for the waveform, pulse width and intensities are performed by the setting means 101 (S41). Thereafter, in response to this command, the stimulus parameter setting means 107 is actuated to read out the frequencies from the ROM 34. These frequencies are output via the output port 36 to the waveform generating section 41 and voltage stimulus generating circuit 51 (S42). These frequencies may be fixed values, or variable at random. Thereafter, the electrical stimulus signal is similarly controlled as in the previous frequency modulation (S43). Subsequently, based upon the modulation system selected by the above-described modulation system setting means 106, the command for alternately outputting the current and voltage stimulus signals at a constant rate, or random rate, is given to the control system selecting section 60 by this setting means 106. Accordingly, these stimulus signals are alternately output (S44), which enables the voltage and current stimulus to be alternately applied to a biological body.

The above alternate selection can be performed by the command from the input section 20 and the stimulus signals are read out and output from the ROM 34. These processes can be performed by the setting means 101.

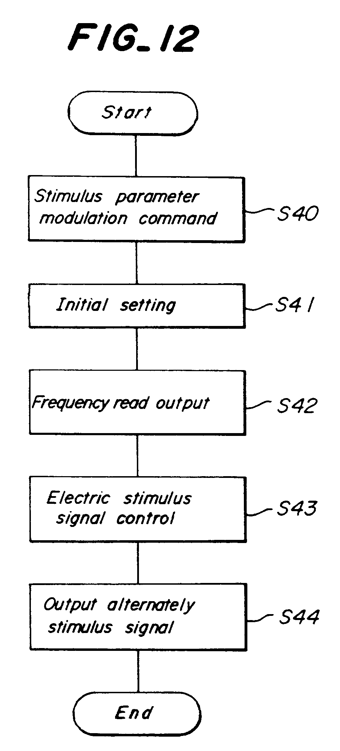

Secondly, the situation will be described in which the intensity is variable in stimulus pattern modulation processing. Since this intensity variable process is, in principle similar to the above-described frequency variable process, the frequency as illustrated in Figure 12 is merely substituted by the intensity (amplitude) for the intensity variable process. However, to initialize the frequency, a control signal is sent from the setting means 101 to the PTM 35 and thus, the frequency is automatically locked by this PTM 35. The stimulus pattern modulation is carried out by such an intensity variable system whereby the voltage and current stimulus signals can be alternately applied to the biological body.

In both types of the stimulus pattern modulation processes, the stimulus patterns are controlled by the program previously stored in the CPU 32.

As has been described, the current and voltage stimulus signals are alternately changed in this type of stimulus pattern modulation system. The time period required for this signal change is selected to be from several seconds to several minutes. It is however preferable to select approximately two seconds to one minute.

The reason why such alternation stimulus employing the voltage and current stimulus signals is applied to the biological body is as follows. When, for instance, the current control is performed as a sine wave and an expotential function waveform, and also the voltage control is performed as a rectangular wave and a spike or pin-shaped waveform, no stimulus is given to the biological body during the current control, even if the same current value as in the voltage control method is used. Although the biological body feels no stimulus, it receives electric changes in the current control method, so that this current stimulus method is effective for a biological body under anesthesia, children, or a person who does not like to receive the electrical stimulus signal. However, if only current stimulus causes no sensation or feel to a biological body, it may feel the proper stimulus by alternately changing the voltage and current stimulus.

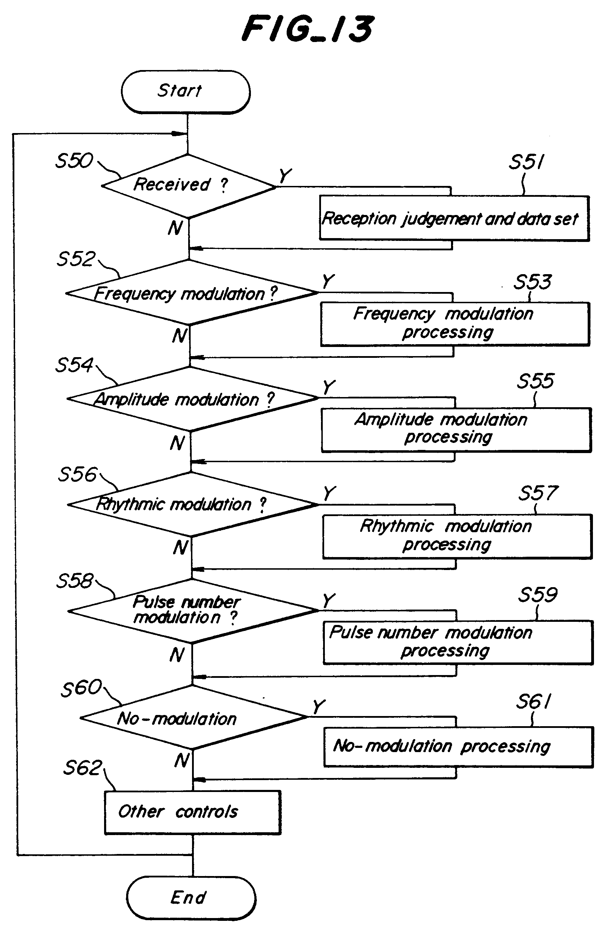

Figure 13 shows one example of a main control flow of apparatus embodying the present invention.

First, a check is made whether or not the signal sent from the input section 20 is received (S50). If the input signal is received, then the subsequent frequency processing operation is performed after the data used for the above judgement and also each of the data have been set. If such an input signal is not yet received, the subsequent frequency processing operation is similarly carried out.

Thereafter, a judgement is made whether or not the frequency modulation is performed (S52). If its command is made, then the control advances to the next processing step after the frequency modulation processing has been carried out (S53). If no command is made, the control directly advances to the succeeding process.

Thereafter, a judgement is made whether or not amplitude modulation is to be performed (S54). If its command is made, the control advances to the succeeding step after the amplitude modulation processing has been accomplished (S55). If no command is made, the control directly advances to the subsequent step.

Subsequently, a judgement is made whether or not rhythmic modulation is to be done (S56). If the command is made, then the control advances to the next processing step after the rhythmic modulation has been executed (S57). If no, then the control advances directly to the subsequent processing step.

Next, a judgement is made whether or not pulse number modulation is to be done (S58). If the command is made, the control advances to the subsequent processing step after the frequency modulation process has been performed (S59). If there is no command, the control directly advances to the next processing step.

Then, a judgement is made whether any modulation processing is to be done (S60). If no modulation command is made, the control advances to the next processing step after no modulation processing has been performed (S61). Otherwise, the control advances directly to the succeeding process step.

Then, in addition, a predetermined process such as a stimulus pattern process is controlled (S62).

It should be noted that these signal processings are repeated during a series of operations of the low frequency stimulus signal generating apparatus. The judgement processings for judging the sorts of various modulations are performed in the modulation system setting means 106 in the CPU 32. The judgement results are sent to the respective frequency forcibly converting means 103, cure intensity setting means 104, pulse number setting means 105, and stimulus parameter setting means 107. Then, the desired signal process is effected.

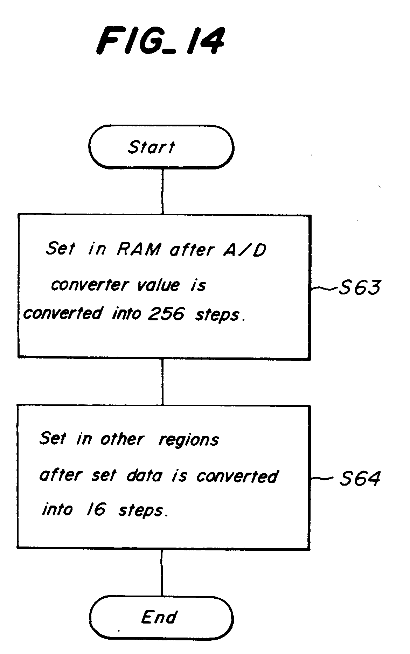

In Figure 5, the sound volume level data which has been input from the A/D converter 92 into the CPU 32, is converted into 8-bit data and set into the RAM 33 by means of the converting means 102 as illustrated in the flowchart of Figure 14 (S63). This data conversion is carried out in such a manner that the data value derived from the A/D converter 92 is converted into the 8-bit data having 256 steps. This set data is further converted into 16-stepped data and set into another field in the RAM 33 (S64).

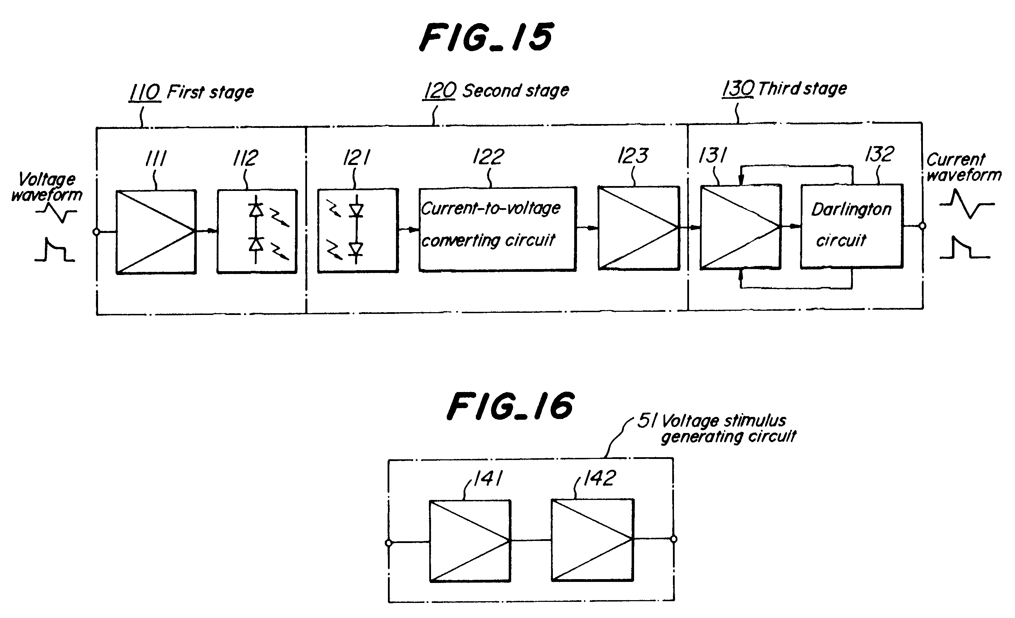

A brief explanation will now be made of the current stimulus generating circuit 47 and the voltage stimulus generating circuit 51 (which have previously been described in outline as shown in Figure 2) with reference to Figures 15 and 16.

It should be noted that both generating circuits 47 and 51 can be assembled by employing conventional electronic circuit technology.

In Figure 15, there is shown a block diagram of one example of the current stimulus signal generating circuit. As previously described, this circuit is operable to output a current having the same waveform as that of the input voltage.

This generating circuit may comprise a first stage 110, a second stage 120 and a third stage 130, including, for instance, an operational amplifier 111, a photocoupler 112, 121 and a darlington circuit 132.

The first stage 110 includes the operational amplifier 111 and a light emitting diode 112 of the photocoupler to convert the voltage waveform into the current waveform at a constant voltage level, and to convert the input voltage into a photo-output.

The second stage 120 includes a light receiving element 121 of the photocoupler, a current-to-voltage converting circuit 122 for converting the current supplied from the light receiving element 121 into a voltage, and an operational amplifier 123 for amplifying the resultant voltage, so as to produce an amplified voltage.

The third stage 130 produces the output current by amplifying the voltage with an operational amplifier 131 and thereafter, by converting the amplified voltage into a current in the darlington circuit 132. In this case, the circuit arrangement is designed so that even if the impedance of the biological body to which this output current is provided is varied, a current having the same waveform as that of the voltage can be continuously output by feeding back the output of the darlington circuit 132 to the operational amplifier 131.

It should be noted that the circuit arrangement of the current stimulus signal generating circuit 46 is not limited to the above embodiment, but may be modified.

The voltage stimulus generating circuit is a circuit for simply amplifying the input voltage, a suitable circuit arrangement for which is shown in Figure 16. This voltage stimulus generating circuit 51 comprises an operational amplifier 141 and a push-pull amplifier 142 for amplifying the output from the operational amplifier, so as to output a voltage which is obtained by converting the amplitude and frequency of the input voltage into a predetermined voltage.

Similarly, the generating circuit 51 is not limited to the above-described circuit arrangement, but may be modified.

The control method and/or apparatus are not restricted to the above-described method and circuit arrangement, but may be modified, for example as follows.

For instance, the arrangement of the apparatus as illustrated in Figure 2 may be constructed by employing other circuit arrangements. Similarly, the operation method may be modified.

It is also possible to generate the electrical stimulus signal by combining more than two modulations selected from the above-described frequency modulation, amplitude modulation, pulse modulation, rhythmic modulation, and stimulus pattern modulation (alternate stimulus).

As has been described in detail, the low frequency electrical stimulus in accordance with the sound volume (sound pressure) levels of music or other sounds can be given to a biological body in substantially real time by alternately changing the current or voltage control system. As a result, improved curative effects as well as pain relaxation effects can be achieved, compared with the previously-proposed cure systems based on the 1/f fluctuation theory.

Particularly in the case of rhythm modulation, the current or voltage stimulus signal is controlled based upon the sound waveforms when the sound information is input. When no sound information is present, these voltage and current stimulus signals are controlled based upon a quasi-sound waveform. Then, since one of these stimulus signals can be output as the electrical stimulus signal in real time, pain reduction and curing effects can be improved compared with the previously-proposed 1/f fluctuation theory. In addition, since the present arrangement utilizes only sound information containing low frequencies, typically lower than approximately 200 Hz (such as bass and drums in music), unpleasant feelings can be effectively reduced or eliminated by producing a rhythmic feeling.

Furthermore, particularly in the case where the current stimulus signal and voltage stimulus signal are alternately switched-over to output as the electrical stimulus signal, since a biological body can be electrically stimulated in real time in accordance with the tempo of the favourable music, enhanced curing effects and pain reduction can be realized in the field of the musical curing method, as compared with the previously-proposed musical curing method employing 1/f fluctuation theory. Furthermore, as the current and voltage control systems are alternately switched to give such alternating stimulus to the biological body, it is possible to avoid the absence of stimulus feeling for the biological body. Accordingly, lack of sensation or feeling can be eliminated, pain effects can be reduced and the curative effects can be improved. Moreover, since a low frequency electrical stimulus signal is employed, relaxation can be expected both physically and mentally.

## Claims

1. Low frequency electrical stimulus signal generating apparatus for generating a stimulus signal for a patient from a sound source (16) such that the patient may simultaneously hear sound from the sound source (16) and feel the stimulus signal, the apparatus being characterised by:

an input section (20) for setting and adjusting operating conditions of the apparatus;

a waveform control section (80) operable to output a signal with a low frequency component of the sound from the sound source (16);

a control section (30) operable to sample the sound volume level from the sound source (16) at a rate between 2 and 100 Hz, and to convert the sampled sound levels into respective frequencies below 60 Hz; and

a current and/or voltage control section (40,50) responsive to a selected one of the low frequency sound component and the respective frequencies from the control section (30) depending on the setting of the input section (20) to produce current and/or voltage stimulus signals modulated by the selected one of the low frequency sound component and the respective frequencies.

2. Apparatus according to claim 1, including a control system selection section (60) for switching only one of said current and voltage stimulus signals based upon control information derived from said control section (30) and for outputting the same as the electrical stimulus signal.

3. Apparatus according to claim 1 or claim 2, wherein the current control section (40) includes:

a waveform generating section (41) for outputting a voltage waveform signal corresponding to said sound volume levels; and

a current stimulus signal generating circuit (47) for outputting a current stimulus signal having the same waveform as that of the voltage waveform signal from the waveform generating section (41), when the apparatus is set by the input section (20) in the mode for producing current stimulus signals modulated by the respective frequencies from the control section (30).

4. Apparatus according to claim 1, claim 2 or claim 3, wherein the voltage control section (50) includes:

a waveform generating section (41) for generating a voltage waveform signal having a frequency corresponding to said sound volume levels; and

a voltage stimulus signal generating circuit (51) for outputting the voltage waveform signal from the waveform generating section (41) as a voltage stimulus signal, when the apparatus is set by the input section (20) in the mode for producing voltage stimulus signals modulated by the respective frequencies from the control section (30).

5. Apparatus according to any one of the preceding claims, wherein said control section (30) includes:

means (92) for subdividing said sound volume levels into a plurality of level steps;

a read-only-memory (34) storing a sound volume level-to-frequency conversion table; and

means (32) for reading out from the read-only-memory (34) a frequency corresponding to the sound volume level at each sampling operation.

6. Apparatus according to claim 5, wherein said sound volume level is set to a zero level when said sound volume level is equal to zero or lower than a minimum level.

7. Apparatus according to any one of the preceding claims, wherein said waveform control section (80) is operable, in the absence of sound from said sound source (16), to generate a periodically-repeating waveform.

8. Apparatus according to claim 7, wherein said waveform control section (80) includes:

a comparator (81) for detecting whether or not sound from said sound source (16) is present;

a low pass filter (82) for filtering the sound to produce the low frequency sound component having a frequency lower than approximately 200 Hz;

a rhythm generator (83) for generating the periodically-repeating waveform as quasi-sound information; and

a selection switch (84) for selecting the low frequency sound component when said sound is present, and said quasi-sound information when said sound is absent.

9. Apparatus according to claim 7 or claim 8, wherein:

said current control section (40) includes an intensity setting circuit (46) receiving said low frequency sound component or said quasi-sound information, and a current stimulus signal generating circuit (47) for outputting a current stimulus signal having the same waveform as that of a waveform from said intensity setting circuit (46); and

said voltage control section (50) includes a voltage stimulus generating circuit (51) for outputting a voltage stimulus signal by adjusting said low frequency sound component or said quasi-sound information.

10. Apparatus according to claim 7, claim 8 or claim 9, wherein the intensity of said electrical stimulus signal is selectively adjustable.

11. Apparatus according to any one of the preceding claims, wherein said control section (30) is operable to convert each of the sampled sound levels into a corresponding amplitude control value, and said current and/or voltage control section (40,50) is operable to perform amplitude modulation in response to the amplitude control value within a range of 0 to 100% of an initial set amplitude value of the current and/or voltage stimulus signals, when the apparatus is set by the input section (20) in the mode for producing stimulus signals modulated by the respective frequencies from the control section (30).

12. Apparatus according to claim 11, wherein said control section (30) includes:
means (92) for subdividing the sound volume levels into a plurality of level steps; and
means (36) for converting the subdivided levels into the corresponding amplitude control values and for outputting the converted control values.

13. Apparatus according to claim 11 or claim 12, wherein an electrical stimulus signal having 24 steps is obtained as said amplitude control value within a range between 0 and 100% of the initial set amplitude value.

14. Apparatus according to claim 2, wherein said control information of the current and voltage stimulus signals supplied to the control system selecting section (60) is information which is selected from more than one sort of waveform, frequency and intensity, and said control information is selectable and variable based upon an external input.

15. Apparatus according to claim 14, wherein alternating selection of said current and voltage stimulus signals is performed at random.

16. Apparatus according to claim 15, wherein a time interval of said alternating selection of said current and voltage stimulus signals is set at random within several seconds to several tens of seconds.

17. A method of generating a low frequency electrical stimulus signal for a patient from a sound source (16) such that the patient may simultaneously hear sound from the sound source (16) and feel the stimulus signal, the method being characterised by the steps of:
deriving a low frequency component signal of the sound from the sound source (16);
sampling the sound volume level from the sound source (16) at a rate between 2 and 100 Hz;
converting the sampled sound levels into respective frequencies below 60 Hz; and
producing current and/or voltage control stimulus signals modulated by a selected one of the low frequency sound component and the respective frequencies.

18. A method according to claim 17, wherein the respective sound volume levels obtained by said sampling operation are converted into corresponding amplitude control values, and in accordance with said amplitude control values, said current or voltage control is performed by performing amplitude modulation within a range from 0 to 100% of initial setting amplitude values of the current or voltage, when modulation of the stimulus signals by the respective frequencies has been selected.

19. A method according to claim 17 or claim 18, wherein the respective sound volume levels obtained by said sampling operation are converted into pulse number control values, and in accordance with said pulse number control values, pulse number modulation to the current or voltage is carried out, when modulation of the stimulus signals by the respective frequencies has been selected.

20. A method according to claim 19, wherein said pulse number modulation is performed in such a manner that the pulse number is within a range from 0 to 30 in accordance with said sound volume levels.

21. A method according to any one of claims 17 to 20, wherein presence of sound from said sound source (16) is detected, the low frequency sound component has a frequency lower than approximately 200 Hz, quasi-sound information derived from a periodically-repeating waveform is generated in the absence of detected sound, and the waveforms of said low frequency sound component and said quasi-sound information are selectively utilized to enable said current and/or voltage stimulus signals to be controlled.

22. A method according to any one of claims 17 to 21, wherein the intensity of said stimulus signal is selectively adjustable.

23. A method according to any one of claims 17 to 22, wherein control information previously stored in a control section (30) may be used selectively instead of sound from the sound source (16) so as to control said current and voltage stimulus signals, and said current and voltage stimulus signals are alternately output as the electrical stimulus signal.

24. A method according to claim 23, wherein said control information of the current and voltage stimulus signals is information which is selected from more than one sort of waveform, frequency and intensity, and said control information is selectable and variable based upon an external input.

25. A method according to claim 23 or claim 24, wherein the alternating selection of said current and voltage stimulus signals is performed at random.

26. A method according to claim 25, wherein a time interval of said alternating selection of said current and voltage stimulus signals is set at random within several seconds to several tens of seconds.

**Patentansprüche**

1. Vorrichtung zur Erzeugung eines niederfrequenten elektrischen Reizsignals für die Erzeugung eines Reizsignales für einen Patienten von einer Schallquelle (16), derart, daß der Patient gleichzeitig einen Ton bzw. Schall aus der Schallquelle (16) hört und das Reizsignal fühlt, wobei die Vorrichtung gekennzeichnet ist durch
einen Eingabeabschnitt (20) für das Festsetzen und Einstellen von Betriebsbedingungen für die Vorrichtung,
einen Wellenformregelabschnitt (80), der so betreibbar ist, daß er ein Signal mit einen niederfrequenten Komponente des Schalles aus der Schallquelle (16) ausgibt,
einen Regelabschnitt (30), der so betreibbar ist, daß er den Schallautstärkepegel der Schallquelle (16) mit einer Rate zwischen 2 und 100 Hz abtastet, und daß er die abgetasteten Schallpegel in entsprechende Frequenzen unterhalb von 60 Hz umwandelt, und
einen Strom- und/oder Spannungsregelabschnitt (40, 50), der auf eine ausgewählte Komponente des niederfrequenzen Schalls und die entsprechenden Frequenzen von dem Regelabschnitt (30) anspricht in Abhängigkeit von der Einstellung des Eingabeabschnittes (20), um Strom- und/oder Spannungsreizsignale zu erzeugen, die durch die ausgewählte, niederfrequenze Schallkomponente und die entsprechenden Frequenzen moduliert sind.

2. Vorrichtung nach Anspruch 1, einschließlich eines Regelsystemauswahlabschnittes (60) zum Umschalten nur eines der Strom- und Spannungsreizsignale auf der Basis einer Regelinformation, die aus dem Regelabschnitt (30) abgeleitet wird, und für die Ausgabe desselben als das elektrische Reizsignal.

3. Vorrichtung nach Anspruch 1 oder 2, wobei der Stromregelabschnitt (40) aufweist:
einen Wellenformerzeugungsabschnitt (41) für die Ausgabe eines Spannungswellenformsignales, welches den Schallpegelwerten entspricht, und
einen Erzeugungsschaltkreis (47) für ein Reizstromsignal für die Ausgabe eines Reizstromsignales, welches dieselbe Wellenform wie das Spannungswellenformsignal von dem Wellenformerzeugungsabschnitt (41) hat, wenn die Vorrichtung durch den Eingabeabschnitt (20) in die Betriebsartzur Erzeugung eines Reizstromsignales eingestellt ist, welches durch die betreffenden Frequenzen von dem Regelabschnitt (30) moduliert wird.

4. Vorrichtung nach Anspruch 1, 2 oder 3, wobei der Spannungsregelabschnitt (50) aufweist: einen Wellenformerzeugungsabschnitt (41) für die Erzeugung eines Spannungswellenformsignales, welches eine Frequenz hat, die den Schallpegelwerten entspricht, und einen Erzeugungsschaltkreis (41) für ein Reizspannungssignal für die Ausgabe eines Spannungswellenformsignales aus dem Wellenformerzeugungsabschnitt (41) als ein Reizspannungssignal, wenn die Vorrichtung durch den Eingabeabschnitt (20) in die Betriebsart für das Erzeugen von Reizspannungssignalen eingestellt ist, die durch die betreffenden Frequenzen von dem Regelabschnitt (30) moduliert werden.

5. Vorrichtung nach einem der vorstehenden Ansprüche, wobei der Regelabschnitt (30) aufweist:
Einrichtungen (92) für das Unterteilen der Schallpegelwerte in eine Mehrzahl von Pegelschritten,
einen Nur-Lese-Speicher (34) (Read-Only-Memory), welcher eine Umwandlungstabelle von Schallpegelwerten zu Frequenzen speichert, und
Einrichtungen (32) für das Auslesen einer Frequenz aus dem Nur-Lese-Speicher (34), welche dem Schallpegelwert bei jedem Abtastvorgang entspricht.

6. Vorrichtung nach Anspruch 5, wobei der Schallpegelwert auf ein Nullniveau gesetzt wird, wenn der Schallpegelwert Null ist oder kleiner als ein Minimalwert ist.

7. Vorrichtung nach einem der vorstehenden Ansprüche, wobei der Wellenformregelabschnitt (80) bei Abwesenheit von Schall von der Schallquelle (60) so betreibbar ist, daß er eine sich periodisch wiederholende Wellenform erzeugt.

8.  Vorrichtung nach Anspruch 7, wobei der Wellenformregelabschnitt (80) aufweist:
einen Vergleicher (81), um zu erfassen, ob Schall von der Schallquelle (16) vorhanden ist oder nicht,
einen Tiefpaßfilter (82) für das Filtern des Schalles, um die Niederfrequenzschallkomponente herauszufiltern, die eine Frequenz hat, welche niedriger als etwa 200 Hz ist, einen Rhythmusgenerator (83) für die Erzeugung der sich periodisch wiederholenden Wellenform als eine Quasi-Schallinformation, und
einen Wahlschalter (84) für die Auswahl der niederfrequenten Schallkomponente, wenn der Schall bzw. Ton vorhanden ist, und der Quasi-Schallinformation, wenn der Schall nicht vorhanden ist.

9.  Vorrichtung nach Anspruch 7 oder 8, wobei:
der Stromregelabschnitt (40) einen Intensitätseinstellschaltkreis (46) aufweist, welcher die niederfrequente Schallkomponente oder die Quasi-Schallinformation empfängt, und einen das Reizstromsignal erzeugenden Schaltkreis (47) aufweist, um ein Reizstromsignal abzugeben, welches dieselbe Wellenform hat wie die von dem Intensitätseinstellschaltkreis (46), und wobei
der Spannungsregelabschnitt (50) einen eine Reizspannung erzeugenden Schaltkreis (41) aufweist, um ein Reizspannungssignal abzugeben durch Einstellen der niederfrequenten Schallkomponente oder der Quasi-Schallinformation.

10. Vorrichtung nach Anspruch 7, 8 oder 9, wobei die Intensität des elektrischen Reizsignals wahlweise einstellbar ist.

11. Vorrichtung nach einem der vorstehenden Ansprüche, wobei der Regelabschnitt (30) so betreibbar ist, daß er jeden der abgetasteten Schallpegel in einen entsprechenden Amplitudenregelwert umwandelt und daß der Strom und/oder Spannungsregelabschnitt (40, 50) so betreibbar ist, daß er eine Amplitudenmodulation unter Ansprechen auf den Amplitudenregelwert innerhalb eines Bereiches von 0 bis 100 % eines anfänglich eingestellten Amplitudenwertes der Reizstrom- und/oder Reizspannungssignale durchführt, wenn die Vorrichtung durch den Eingabeabschnitt (20) in die Betriebsart für das Erzeugen von Reizsignalen eingestellt ist, welche durch die jeweiligen Frequenzen von dem Regelabschnitt (30) moduliert werden.

12. Vorrichtung nach Anspruch 11, wobei der Regelabschnitt (30) aufweist:
Einrichtungen (92) zum Aufteilen der Schallpegelwerte in eine Mehrzahl von Pegelschritten, und
Einrichtungen zum Umwandeln der aufgeteilten Pegel in entsprechende Amplitudenregelwerte sowie für die Ausgabe der umgewandelten Regel- bzw. Steuerwerte.

13. Vorrichtung nach Anspruch 11 oder 12, wobei als der Amplitudenregelwert innerhalb eines Bereiches zwischen 0 und 100 % des anfänglich eingestellten Amplitudenwertes ein elektrisches Reizsignal mit 24 Schritten erhalten wird.

14. Vorrichtung nach Anspruch 2, wobei die Regelinformation der Reizstrom- und Reizspannungssignale, welche dem Regelsystemauswahlabschnitt (60) zugeführt werden, Information ist, welche aus mehr als einer Art von Wellenform, Frequenz und Intensität ausgewählt wird, und daß die Regelinformation auf der Basis einer externen Eingabe auswählbar und variabel ist.

15. Vorrichtung nach Anspruch 14, wobei eine abwechselnde Auswahl der Reizstrom- und Reizspannungssignale nach dem Zufallsprinzip durchgeführt wird.

16. Vorrichtung nach Anspruch 15, wobei ein Zeitintervall der abwechselnden Auswahl der Reizstrom- und Reizspannungssignale nach dem Zufallsprinzip innerhalb einiger Sekunden bis zu mehreren -zig Sekunden eingestellt wird.

17. Verfahren zum Erzeugen eines niederfrequenten elektrischen Reizsignales für einen Patienten von einer Schallquelle (16), derart, daß der Patient gleichzeitig Schall von der Schallquelle (16) hören und das Reizsignal fühlen kann, wobei das Verfahren durch die Schritte gekennzeichnet ist:
Ableiten einer niederfrequenten Signalkomponente aus dem Schall von der Schallquelle (16),
Abtasten des Schallautstärkepegels von der Schallquelle (16) mit einer Rate zwischen 2 und 100 Hz,
Umwandeln der abgetasteten Schallpegel in entsprechende Frequenzen unterhalb von 60 Hz, und

Erzeugen von Reizstrom- und/oder Reizspannungsregelsignalen, welche nach Wähl moduliert werden durch die niederfrequente Schallkomponente oder die entsprechenden Frequenzen.

18. Verfahren nach Anspruch 17, wobei die entsprechenden Schallautstärkepegel, welche durch den Abtastvorgang erhalten werden, in entsprechende Amplitudenregelwerte umgewandelt werden, und wobei entsprechend den Amplitudenregelwerten die Strom- oder Spannungsregelung durchgeführt wird, indem innerhalb eines Bereiches von 0 bis 100 % von anfänglich eingestellten Amplitudenwerten des Stromes oder der Spannung eine Amplitudenmodulation durchgeführt wird, wenn die Modulation der Reizsignale durch die entsprechenden Frequenzen ausgewählt worden ist.

19. Verfahren nach Anspruch 17 oder 18, wobei die entsprechenden Schallautstärkepegel, die durch den Abtastvorgang erhalten werden, in Impuls- bzw. Pulszahlregelwerte umgewandelt werden und daß entsprechend den Pulszahlregelwerten eine Pulszahlmodulation des Stromes oder der Spannung durchgeführt wird, wenn die Modulation der Reizsignale durch die entsprechenden Frequenzen ausgewählt worden ist.

20. Verfahren nach Anspruch 19, wobei die Pulszahlmodulation derart durchgeführt wird, daß die Pulszahl innerhalb eines Bereiches von 0 bis 30 in Übereinstimmung mit den Schallautstärkepegeln liegt.

21. Verfahren nach einem der Ansprüche 17 bis 20, wobei das Vorhandensein von Schall aus der Schallquelle (16) erfaßt wird, die niederfrequente Schallkomponente eine Frequenz hat, die niedriger als etwa 200 Hz ist, bei Abwesenheit von erfaßtem Schall eine Quasi-Schallinformation von einer sich periodisch wiederholenen Wellenform erzeugt wird, und die Wellenformen der niederfrequenten Schallkomponente und der Quasi-Schallinformation wahlweise verwendet werden, um die zu regelnden Reizstrom- und/oder Reizspannungssignale einzuschalten.

22. Verfahren nach einem der Ansprüche 17 bis 21, wobei die Intensität des Reizsignales wahlweise einstellbar ist.

23. Verfahren nach einem der Ansprüche 17 bis 22, wobei die Regelinformation, die zuvor in einem Regelabschnitt (30) gespeichert wurde, wahlweise anstelle des Schalles aus der Schallquelle (60) verwendet werden kann, um so die Reizstrom- und Reizspannungssignale zu regeln, und wobei die Reizstrom- und Reizspannungssignale abwechselnd als das elektrische Reizsignal ausgegeben werden.

24. Verfahren nach Anspruch 23, wobei die Regelinformation der Reizstrom- und Reizspannungssignale eine Information ist, welche aus mehr als einer Sorte von Wellenformen, Frequenz und Intensität ausgewählt wird, und daß die Regelinformation auf der Basis einer externen Eingabe auswählbar und variabel ist.

25. Verfahren nach Anspruch 23 oder Anspruch 24, wobei die abwechselnde Auswahl der Reizstrom- und Reizspannungssignale nach dem Zufallsprinzip durchgeführt wird.

26. Verfahren nach Anspruch 25, wobei für die abwechselnde Auswahl der Reizstrom- und Reizspannungssignale ein Zeitintervall auf einen Zufallswert innerhalb mehrerer Sekunden bis hin zu mehreren -zig Sekunden eingestellt wird.

**Revendications**

1. Appareil de génération de signal de stimulation électrique, à basse fréquence, pour générer un signal de stimulation pour un patient, à partir d'une source de son (16), tel que le patient peut simultanément entendre du son à partir de la source de son (16) et sentir le signal de stimulation, l'appareil étant caractérisé par :

    une section d'entrée (20) pour établir et ajuster des conditions de fonctionnement de l'appareil ;

    une section de commande de forme d'onde (80) prête à être mise en service afin d'émettre un signal avec une composante du son à basse fréquence, à partir de la source de son (16) ;

    une section de commande (30) prête à être mise en service afin d'échantillonner le niveau de volume de son, a partir de la source de son (16), à un taux entre 2 et 100 Hz, et afin de convertir les niveaux de son échantillonnés en des fréquences respectives inférieures à 60 Hz ; et

    une section de commande de courant et/ou de tension (40, 50) réagissant à une composante de son à basse fréquence sélectionnée, et aux fréquences respectives, à partir de la section de commande (30), en fonction de l'établissement de la section d'entrée (20), afin de produire des signaux de stimulation de courant et/ou de tension, modulés par la composante de son à basse fréquence sélectionnée précitée et les fréquences respectives.

2. Appareil selon la revendication 1, incluant une section de sélection de système de commande (60), pour commuter seulement un desdits signaux de stimulation de courant et de tension, sur la base d'information de commande, dérivée à partir de ladite section de commande (30), et, pour émettre le même signal que le signal de stimulation électrique.

3. Appareil selon la revendication 1 ou la revendication 2, dans lequel la section de commande de courant (40) inclut :

une section de génération de forme d'onde (41) pour émettre un signal de forme d'onde de tension correspondant auxdits niveaux de volume de son ; et

un circuit de génération de signal de stimulation de courant (47) pour émettre un signal de stimulation de courant ayant la même forme d'onde que celle du signal de forme d'onde de tension, à partir de la section de génération de forme d'onde (41), lorsque l'appareil est établi, par la section d'entrée (20), dans le mode pour produire des signaux de stimulation de courant, modulés par les fréquences respectives, à partir de la section de commande (30).

4. Appareil selon la revendication 1, la revendication 2 ou la revendication 3, dans lequel la section de commande de tension (50) inclut :

une section de génération de forme d'onde (41) pour générer un signal de forme d'onde de tension ayant une fréquence correspondant auxdits niveaux de volume de son ; et

un circuit de génération de signal de stimulation de tension (51) pour émettre le signal de forme d'onde de tension, à partir de la section de génération de forme d'onde (41), comme un signal de stimulation de tension, lorsque l'appareil est établi, par la section d'entrée (20), dans le mode pour produire des signaux de stimulation de tension, modulés par les fréquences respectives, à partir de la section de commande (30).

5. Appareil selon une quelconque des précédentes revendications, dans lequel ladite section de commande (30) inclut :

des moyens (92) pour subdiviser lesdits niveaux de volume de son en une pluralité de degrés de niveau ;

une mémoire pour lecture seulement (34) emmagasinant une table de conversion niveau de volume de son-en-fréquence ; et

des moyens (32) pour sortir par lecture à partir de la mémoire pour lecture seulement (34) une fréquence correspondant au niveau de volume de son, à chaque opération d'échantillonnage.

6. Appareil selon la revendication 5, dans lequel ledit niveau de volume de son est établi à un niveau zéro, lorsque ledit niveau de volume de son est égal à zéro ou inférieur à un niveau minimum.

7. Appareil selon une quelconque des précédentes revendications, dans lequel ladite section de commande de forme d'onde (80) est prête à être mise en service, en l'absence de son à partir de ladite source de son (16), afin de générer une forme d'onde se répétant périodiquement.

8. Appareil selon la revendication 7, dans lequel ladite section de commande de forme d'onde (80) inclut :

un comparateur (81) pour détecter si oui ou non du son, à partir de la source de son (16), est présent ;

un filtre passe-bas (82) pour filtrer le son, afin de produire la composante de son à basse fréquence ayant une fréquence inférieure à approximativement 200 Hz ;

un générateur de rythme (83) pour générer la forme d'onde se répétant périodiquement comme information quasi sonore ; et

un commutateur de sélection (84) pour sélectionner la composante de son à basse fréquence, lorsque ledit son est présent, et ladite information quasi sonore, lorsque ledit son est absent.

9. Appareil selon la revendication 7 ou la revendication 8, dans lequel :

ladite section de commande de courant (40) inclut un circuit d'établissement d'intensité (46) recevant ladite composante de son à basse fréquence ou ladite information quasi sonore, et un circuit de génération de signal de stimulation de courant (47), pour émettre un signal de stimulation de courant ayant la même forme d'onde que celle d'une forme d'onde à partir dudit circuit d'établissement d'intensité (46) ; et

ladite section de commande de tension (50) inclut un circuit de génération de stimulation de tension (51), pour émettre un signal de stimulation de tension en ajustant ladite composante de son à basse fréquence ou ladite information quasi sonore.

10. Appareil selon la revendication 7, la revendication 8 ou la revendication 9, dans lequel l'intensité dudit signal de stimulation électrique est ajustable sélectivement.

11. Appareil selon une quelconque des précédentes revendications, dans lequel ladite section de commande (30) est prête à être mise en service afin de convertir chacun des niveaux de son échantillonnés en une valeur de commande d'amplitude correspondante, et ladite section de commande de courant et/ou de tension (40, 50) est prête à être mise en service, afin d'effectuer une modulation d'amplitude en réponse à la valeur de commande d'amplitude, à l'intérieur d'une plage de 0 à 100 % d'une valeur d'amplitude établie initialement des signaux de stimulation de courant et/ou de tension, lorsque l'appareil est établi, par la section d'entrée (20), dans le mode pour produire des signaux de stimulation modulés par les fréquences respectives, à partir de la section de commande (30).

12. Appareil selon la revendication 11, dans lequel ladite section de commande (30) inclut :

des moyens (92) pour subdiviser les niveaux de volume de son en une pluralité de degrés de niveau ; et

des moyens (36) pour convertir les niveaux subdivisés en les valeurs de commande d'amplitude correspondantes, et pour émettre les valeurs de commande converties.

13. Appareil selon la revendication 11 ou la revendication 12, dans lequel un signal de stimulation électrique ayant 24 degrés est obtenu comme dite valeur de commande d'amplitude, à l'intérieur d'une plage entre 0 et 100 % de la valeur d'amplitude établie initialement.

14. Appareil selon la revendication 2, dans lequel ladite information de commande des signaux de stimulation de courant et de tension, fournie à la section de sélection de système de commande (60), est une information, qui est sélectionnée à partir de plus d'une sorte de forme d'onde, de fréquence et d'intensité, et ladite information de commande est sélectionnable et variable sur la base d'une entrée externe.

15. Appareil selon la revendication 14, dans lequel une sélection alternante desdits signaux de stimulation de courant et de tension est effectuée de façon aléatoire.

16. Appareil selon la revendication 15, dans lequel un intervalle de temps de ladite sélection alternante de signaux de stimulation de courant et de tension est établi de façon aléatoire, à l'intérieur de plusieurs secondes à plusieurs dizaines de secondes.

17. Un procédé de génération d'un signal de stimulation électrique à basse fréquence pour un patient, à partir d'une source de son (16), tel que le patient peut simultanément entendre du son à partir de la source de son (16) et sentir le signal de stimulation, le procédé étant caractérisé par les étapes de :

dérivation d'un signal à composante à basse fréquence du son, à partir de la source de son (16) ;

échantillonnage du niveau de volume de son, a partir de la source de son (16), à un taux entre 2 et 100 Hz ;

conversion des niveaux de son échantillonnés en des fréquences respectives inférieures à 60 Hz ; et

production de signaux de stimulation de commande de courant et/ou de tension, modulés par une composante de son à basse fréquence sélectionnée et les fréquences respectives.

18. Un procédé selon la revendication 17, dans lequel les niveaux de volume de son respectifs, obtenus par ladite opération d'échantillonnage, sont convertis en des valeurs de commande d'amplitude correspondantes, et, en conformité avec lesdites valeurs de commande d'amplitude, ladite commande de courant ou de tension est effectuée en effectuant une modulation d'amplitude, à l'intérieur d'une plage de 0 à 100 % de valeur d'amplitude du courant ou de la tension établies initialement, lorsque la modulation des signaux de stimulation par les fréquences respectives a été sélectionnée.

19. Un procédé selon la revendication 17 ou la revendication 18, dans lequel les niveaux de volume de son respectifs, obtenus par ladite opération d'échantillonnage, sont convertis en des valeurs de commande de nombre d'impulsions, et, en conformité avec lesdites valeurs de commande de nombre d'impulsions, une modulation de nombre d'impulsions est exécutée au courant ou à la tension, lorsque la modulation des signaux de stimulation par les fréquences respectives a été sélectionnée.

20. Un procédé selon la revendication 19, dans lequel ladite modulation de nombre d'impulsions est effectuée de telle manière, que le nombre d'impulsions est à l'intérieur d'une plage de 0 à 30, en conformité avec lesdits niveaux de volume de son.

**21.** Un procédé selon une quelconque des revendications 17 à 20, dans lequel la présence de son à partir de ladite source de son (16) est détectée, la composante de son à basse fréquence a une fréquence inférieure à approximativement 200 Hz, une information quasi sonore, dérivée à partir d'une forme d'onde se répétant périodiquement, est générée en l'absence de son détecté, et les formes d'onde de ladite composante de son à basse fréquence et de ladite information quasi sonore sont sélectivement utilisées afin de rendre aptes lesdits signaux de stimulation de courant et/ou de tension à être commandés.

**22.** Un procédé selon une quelconque des revendications 17 à 21, dans lequel l'intensité dudit signal de stimulation est ajustable sélectivement.

**23.** Un procédé selon une quelconque des revendications 17 à 22, dans lequel de l'information de commande, préalablement emmagasinée dans une section de commande (30), peut être utilisée sélectivement à la place de son à partir de la source de son (16), de façon à commander lesdits signaux de stimulation de courant et de tension, et lesdits signaux de stimulation de courant et de tension sont émis alternativement comme le signal de stimulation électrique.

**24.** Un procédé selon la revendication 23, dans lequel ladite information de commande des signaux de stimulation de courant et de tension est une information, qui est sélectionnée à partir de plus d'une sorte de forme d'onde, de fréquence et d'intensité, et ladite information de commande est sélectionnable et variable sur la base d'une entrée extérieure.

**25.** Un procédé selon la revendication 23 ou la revendication 24, dans lequel la sélection alternante desdits signaux de stimulation de courant et de tension est effectuée de façon aléatoire.

**26.** Un procédé selon la revendication 25, dans lequel un intervalle de temps de ladite sélection alternante desdits signaux de stimulation de courant et de tension est établi de façon aléatoire, à l'intérieur de plusieurs secondes à plusieurs dizaines de secondes.

FIG.1

# FIG.2A

20 Input section

Intensity
Waveforms
Controlling system
Selection of electrodes

Frequency

Stimulus pattern

Volume level — a

A/D Converter

Rhythm stimulus — b

92

Interface

31

CPU

32

RAM

33

ROM

34

35

PTM

36

Output port

37

Input port

97

96

Pulse number generator

c

d

e

f

g

h

i

j

30 Control section

10 Stimulus signal generating section

# FIG_2B

10 Stimulus signal generating section

91 Sound volume-to-DC level converter

80 Waveform control section

81 Comparator

84

82 Low-pass filter

Output port

Rhythm-modulated waveform

Rhythm generator

83

16 Sound source device

90 AMP

93 Electronic volume

94 AMP

95

SW

22 DC Converter

23 LED

21 Display section

Output port

Output port

43 Frequency clock section

42 Waveform selecting section

46 47 Intensity setting circuit

Current stimulus generating circuit

45 Waveform generating circuit

44 Pulse width data section

Waveform select

41 Waveform generating section

Intensity

Waveform

Voltage stimulus generating circuit

Frequency 51

Controlling system selecting section

60

70 Output section

40 Current control section

50 Voltage control section

a b c d e f g h i j

24

FIG.3A

FIG.3B

FIG.3C

FIG.3D

FIG.3E

FIG.3F

FIG.3G

FIG.3H

FIG.3I

FIG.3J

FIG.3K

EP 0 268 366 B1

# FIG_4

Table conversion

| 0 | 1 | 2 | 3 | 4 | 5 | 6 | 7 | 8 | 9 | 10 | 11 | 12 | 13 | 14 | 15 | Step |
|-----|---|-----|---|---|---|---|---|---|---|----|----|----|----|----|----|------|
| 1.5 | 2 | 2.5 | 3 | 4 | 5 | 6 | 7 | 8 | 9 | 10 | 15 | 17 | 22 | 27 | 30 | Hz |

# FIG_5

# FIG_6

```
   ┌──────────┐
   │  Start   │
   └────┬─────┘
        │
   ┌────▼──────────┐
   │ Process input │──── S1
   └────┬──────────┘
        │
   ┌────▼──────────┐
   │ Output control│──── S2
   │  information  │
   └────┬──────────┘
        │
   ┌────▼─────┐
   │   End    │
   └──────────┘
```

# FIG_7

```
   ┌──────────┐
   │  Start   │
   └────┬─────┘
        │
   ┌────▼──────────┐
   │ Cure starting │──── S3
   │     data      │
   └────┬──────────┘
        │
   ┌────▼──────────┐
   │   Sampling    │──── S4
   └────┬──────────┘
        │
   ┌────▼──────────┐
   │ Write in RAM  │──── S5
   └────┬──────────┘
        │
   ┌────▼──────────┐
   │   Read out    │──── S6
   │  from RAM     │
   └────┬──────────┘
        │
   ┌────▼──────────┐
   │ Convert into  │──── S7
   │   16 steps    │
   └────┬──────────┘
        │
   ┌────▼──────────┐
   │Table converting│──── S8
   │   ( ROM )     │
   └────┬──────────┘
        │
   ┌────▼──────────┐
   │Output frequency│──── S9
   └────┬──────────┘
        │
   ┌────▼─────┐
   │   End    │
   └──────────┘
```

# FIG.8

Start

S10 — Is sound volume level lower than 1/16 ?

Y → Set cure intensity to zero — S11

N

S12 — Is present frequency lower than 5 Hz ?

N → Is one cycle accomplished ? — S13

Y

S14 — N — Compare present sound volume with previous sound volume ?

Y

S15 — Convert and set sound pressure level into frequency

S16 — Set cure intensity into set intensity

End

# FIG_9

```
         ┌──────────┐
         │  Start   │
         └────┬─────┘
              │
   ┌──────────────────────────┐
   │  Read out setting intensity │──S20
   └──────────┬───────────────┘
              │
   ┌──────────────────────────┐
   │  Read out soud volume level │──S21
   └──────────┬───────────────┘
              │
   ┌──────────────────────────┐
   │  Calculate cure intensity │──S22
   └──────────┬───────────────┘
              │
         ┌──────────┐
         │   End    │
         └──────────┘
```

# FIG_10

```
         ┌──────────┐
         │  Start   │
         └────┬─────┘
              │
       ┌───────────────┐
       │  Read out RAM  │──S23
       └───────┬───────┘
               │
       ┌───────────────┐
       │ Table conversion│──S24
       │    ( ROM )     │
       └───────┬───────┘
               │
       ┌───────────────┐
       │ Calculate pulse │──S25
       │ number control │
       └───────┬───────┘
               │
         ┌──────────┐
         │   End    │
         └──────────┘
```

# FIG_11

Start

S30 — Rhythmic stimulus command

S31 — Judge whether or not sound information is present

Y

S32 — Sound information waveform → Rhythmic modulation waveform

N

S33 — Quasi-sound waveform → Rhythmic modulation waveform

S34 — Rhythmic modulation waveform selection

S35 — Electric stimulus signal control

End

# FIG. 12

```
        ( Start )
            |
  ┌───────────────────────┐
  │ Stimulus  parameter   │ ── S40
  │ modulation  command   │
  └───────────────────────┘
            |
  ┌───────────────────────┐
  │   Initial  setting    │ ── S41
  └───────────────────────┘
            |
  ┌───────────────────────┐
  │ Frequency read output │ ── S42
  └───────────────────────┘
            |
  ┌───────────────────────┐
  │  Electric  stimulus   │ ── S43
  │   signal  control     │
  └───────────────────────┘
            |
  ┌───────────────────────┐
  │  Output alternately   │ ── S44
  │  stimulus  signal     │
  └───────────────────────┘
            |
        ( End )
```

EP 0 268 366 B1

# FIG_13

```
                    ┌─────────┐
                    │  Start  │
                    └────┬────┘
                         │
      ┌──────────────────┤
      │            S50    │
      │              ╱◇╲
      │         ╱ Received ? ╲───Y───┐        S51
      │          ╲         ╱         │   ┌──────────────────────────────┐
      │              ╲╱              └──▶│ Reception judgement and data set │
      │               │N                 └──────────────────────────────┘
      │            S52 │
      │              ╱◇╲
      │      ╱ Frequency modulation ? ╲──Y──┐      S53
      │       ╲                      ╱      │  ┌──────────────────────────┐
      │            ╲╱                 └─────▶│ Frequency modulation        │
      │             │N                       │      processing            │
      │          S54 │                       └──────────────────────────┘
      │            ╱◇╲
      │    ╱ Amplitude modulation ? ╲──Y──┐        S55
      │     ╲                      ╱      │  ┌──────────────────────────┐
      │          ╲╱                 └─────▶│ Amplitude modulation        │
      │           │N                       │      processing            │
      │        S56 │                       └──────────────────────────┘
      │          ╱◇╲
      │    ╱ Rhythmic modulation ? ╲──Y──┐         S57
      │     ╲                     ╱      │  ┌──────────────────────────┐
      │          ╲╱                └─────▶│ Rhythmic modulation         │
      │           │N                      │      processing            │
      │        S58 │                      └──────────────────────────┘
      │          ╱◇╲
      │    ╱ Pulse number          ╲──Y──┐         S59
      │     ╲ modulation ?        ╱      │  ┌──────────────────────────┐
      │          ╲╱                └─────▶│ Pulse number modulation     │
      │           │N                      │      processing            │
      │        S60 │                      └──────────────────────────┘
      │          ╱◇╲
      │    ╱  No-modulation        ╲──Y──┐         S61
      │     ╲                     ╱      │  ┌──────────────────────────┐
      │          ╲╱                └─────▶│ No-modulation processing    │
      │           │N                      └──────────────────────────┘
      │        S62 │
      │   ┌────────┴────────┐
      │   │  Other controls │
      │   └────────┬────────┘
      └────────────┤
                   │
              ┌────┴────┐
              │   End   │
              └─────────┘
```

33

# FIG_ 14

```
            ┌──────────┐
            │  Start   │
            └────┬─────┘
                 │
      ┌──────────┴──────────┐
      │  Set  in RAM after A/D │
      │  converter value  is   │──── S63
      │ converted into 256 steps.│
      └──────────┬──────────┘
                 │
      ┌──────────┴──────────┐
      │  Set  in other  regions│
      │  after set data is converted │──── S64
      │  into  16  steps.      │
      └──────────┬──────────┘
                 │
            ┌────┴─────┐
            │   End    │
            └──────────┘
```

# FIG_15

110 First stage      120 Second stage      130 Third stage

111   112    121   122   123   131   132

Voltage waveform

Current-to-voltage converting circuit

Darlington circuit

Current waveform

# FIG_16

51 Voltage stimulus generating circuit

141   142

EP 0 268 366 B1